# EUROPÄISCHE PATENTSCHRIFT

(11) **EP 1 322 660 B1**
(45) Veröffentlichungstag und Bekanntmachung des Hinweises auf die Patenterteilung: **30.08.2006**
(21) Anmeldenummer: 01982167.7
(22) Anmeldetag: 28.09.2001
(51) Int. Cl.: C07J 1/00, C07J 71/00, C07J 11/00, A61K 31/565, A61P 5/26

(54) **17ALPHA-FLUORALKYLSTEROIDE, VERFAHREN ZU DEREN HERSTELLUNG UND DIESE VERBINDUNGEN ENTHALTENDE PHARMAZEUTISCHE ZUSAMMENSETZUNGEN**
17ALPHA-FLUOROALKYL STEROIDS, METHOD FOR PRODUCING THE SAME AND PHARMACEUTICAL COMPOSITIONS CONTAINING SAID COMPOUNDS
17ALPHA-FLUORALKYLSTEROIDES, LEUR PROCEDE DE PRODUCTION ET COMPOSITIONS PHARMACEUTIQUES CONTENANT CES COMPOSES

(30) Priorität: 29.09.2000 DE 10049736
(43) Veröffentlichungstag der Anmeldung: 02.07.2003
(73) Patentinhaber: Schering AG, 13353 Berlin-Wedding (DE)
(72) Erfinder: RING, Sven, 07749 Jena (DE); KAUFMANN, Günter, 07743 Jena (DE); WYRWA, Ralf, 07751 Oelknitz (DE); ELGER, Walter, 14195 Berlin (DE)
(86) Internationale Anmeldenummer: PCT/DE2001/003732
(87) Internationale Veröffentlichungsnummer: WO 2002/026759

(56) Entgegenhaltungen:
- WO-A-00/39148
- WO-A-93/13122
- US-A- 3 046 273
- US-A- 3 076 825
- DATABASE CA [Online] CHEMICAL ABSTRACTS SERVICE, COLUMBUS, OHIO, US; BRANDES, STEPHANIE J. ET AL: "Fluorinated androgens and progestins: molecular probes for androgen and progesterone receptors with potential use in positron emission tomography" retrieved from STN Database accession no. 108:16543 XP002191057 & MOL. PHARMACOL. (1987), 32(3), 391-403 ,
- LIU, AIJUN ET AL: "Fluorine-18-labeled androgens: radiochemical synthesis and tissue distribution studies on six fluorine-substituted androgens, potential imaging agents for prostatic cancer" J. NUCL. MED. (1992), 33(5), 724-34 , XP002191054
- BONASERA, THOMAS A. ET AL: "Preclinical evaluation of fluorine-18-labeled androgen receptor ligands in baboons" J. NUCL. MED. (1996), 37(6), 1009-1015, XP002191055
- LIU, AIJUN ET AL: "20-[18F]fluoromibolerone, a positron-emitting radiotracer for androgen receptors: synthesis and tissue distribution studies" J. NUCL. MED. (1991), 32(1), 81-8 , XP000170922
- LIU A ET AL: "SYNTHESIS OF HIGH AFFINITY FLUORINE-SUBSTITUTED LIGANDS FOR THE ANDROGEN RECEPTOR. POTENTIAL AGENTS FOR IMAGING PROSTATIC CANCER BY POSITRON EMISSION TOMOGRAPHY" JOURNAL OF MEDICINAL CHEMISTRY, AMERICAN CHEMICAL SOCIETY. WASHINGTON, US, Bd. 35, Nr. 11, 29. Mai 1992 (1992-05-29), Seiten 2113-2129, XP002009737 ISSN: 0022-2623

## Beschreibung

Die Erfindung betrifft 17-Methylensteroide, Verfahren zu deren Herstellung und diese Verbindungen enthaltende pharmazeutische Zusammensetzungen. Die erfindungsgemäßen Verbindungen besitzen androgene Aktivität.

17-perfluoralkylierte Verbindungen der Estran- und 13-Ethyl-Gonan-Reihe sind bekannt. 17β-Hydroxy-17α-trifluormethyl-estr-4-en-3-on, 17β-Hydroxy-17α-trifluormethyl-estr-4,9-dien-3-on und 17β-Hydroxy-17α-trifluormethyl-estr-4,9,11-trien-3-on, 13-Ethyl-17β-hydroxy-17α-trifluormethyl-gon-4-en-3-on, 13-Ethyl-17β-hydroxy-17α-trifluormethyl-gon-4,9-dien-3-on und 13-Ethyl-17β-hydroxy-17α-trifluormethyl-gon-4,9,11-trien-3-on wurden in Sci. China, Ser. B: Chem. (1997), 40(3), 294-301, CN 94-11218 bzw. Bioorg. Med. Chem. Lett. (1995), 5(17), 1899-1902 beschrieben. Die Verbindungen sollen gestagene Aktivität aufweisen. 17β-Hydroxy-17α-trifluormethyl-androst-4-en-3-on ist als Zwischenprodukt in WO 9313122 beschrieben. 17-Pentafluorethylalkylierte-Steroide der Estran- bzw. Androstanreihe sind bisher nicht bekannt.

Die vorliegende Erfindung stellt 17α-Fluoralkylsteroide der allgemeinen Formel (I) bereit 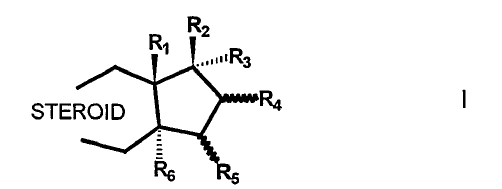 worin R¹ für eine C₁₋₄-Alkylgruppe,
R² für eine Hydroxygruppe, eine Gruppe OC(O)-R²⁰ oder OR²¹ steht, wobei R²⁰ und R²¹ eine C₁₋₁₂-Alkylgruppe, eine C₃₋₈-Cycloalkylgruppe, eine Arylgruppe oder eine Aryl-C₁₋₄₋Alkylgruppe bedeuten ,
R³ für einen Rest der Formel CnF2n+1, wobei n=1, 2, 3, 4, 5 oder 6,
R⁴ und R⁵ jeweils für ein Wasserstoffatom, gemeinsam für eine Doppelbindung oder eine Methylenbrücke,
R⁵ und R⁶ je für ein Wasserstoffatom, gemeinsam für eine Doppelbindung oder eine Methylenbrücke,
STEROID für ein steroidales ABC-Ringsystem der Teilformeln A, B, C, D, E und F stehen: 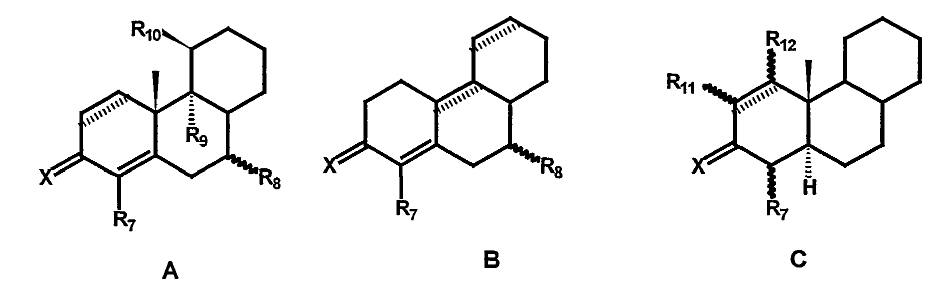 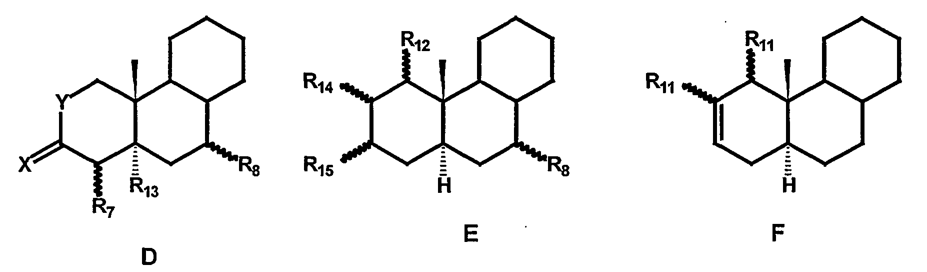 wobei sich in A und C in 1,2-Stellung eine zusätzliche Doppelbindung und sich in B in 8,9 Stellung und 11,12 Stellung eine oder zwei zusätzliche Doppelbindungen befinden können,
R⁷ ein Wasserstoffatom, ein Halogenatom, eine Hydroxylgruppe oder eine Trifluormethylgruppe bedeutet,
X ein Sauerstoffatom oder zwei Wasserstoffatome
R⁸ ein Wasserstoffatom , eine Methyl- oder Ethylgruppe bedeutet,
R⁹ ein Wasserstoffatom oder ein Halogenatom bedeutet oder gemeinsam mit R¹⁰ für eine Doppelbindung steht,
R¹⁰ ein Wasserstoffatom, eine Hydroxylgruppe, eine Methyl- oder Ethylgruppe bedeutet oder gemeinsam mit R⁹ für eine Doppelbindung steht,
R¹¹ ein Wasserstoffatom, eine C₁₋₄-Alkylgruppe, eine Nitrilgruppe, eine
Hydroxymethylen- oder Formylgruppe bedeutet,
R¹² ein Wasserstoffatom, eine C₁₋₄-Alkylgruppe oder eine Nitrilgruppe bedeutet,
R¹¹ und R¹² neben den vorstehend genannten Bedeutungen zusammen eine Methylenbrücke bedeuten,
R¹³ ein Wasserstoffatom oder zusammen mit R⁷ eine Doppelbindung bedeutet,
R¹⁴ und R¹⁵ zusammen für eine Doppelbindung, einen Oxiranring, einen Thiiranring, einen [2,3c]Oxadiazolring, einen [3,2c]Isoxazolring oder einen [3,2c]Pyrazolring stehen,
Y für ein Sauerstoff- oder Stickstoffatom steht,
wobei die geschlängelten Linien an R⁷, R⁸, R¹¹, R¹², R¹³, R¹⁴ und R¹⁵ bedeuten, daß diese Substituenten α- oder β-ständig sein können und die folgenden Verbindungen ausgenommen sind:
17β-Hydroxy-17α-trifluormethyl-androst-4-en-3-on
17β-Hydroxy-17α-trifluormethyl-estr-4-en-3-on,
17β-Hydroxy-17α-trifluormethyl-estr-4,9-dien-3-on,
17β-Hydroxy-17α-trifluormethyl-estr-4,9,11-trien-3-on,
13-Ethyl-17β-hydroxy-17α-trifluormethyl-gon-4-en-3-on,
13-Ethyl-17β-hydroxy-17α-trifluormethyl-gon-4,9-dien-3-on und
13-Ethyl-17β-hydroxy-17α-trifluormethyl-gon-4,9,11-trien-3-on.

Die erfindungsgemäßen Verbindungen besitzen androgene Aktivität.

Unter "C₁₋₄-Alkylgruppe" wird im Sinne der vorliegenden Erfindung ein verzweigter oder geradkettiger Alkylrest mit 1 bis 4 Kohlenstoffatomen verstanden. Als Beispiele seien eine Methyl-, Ethyl-, n-Propyl-, i-Propyl-, n-Butyl-, i-Butyl- oder tert.-Butylgruppe genannt.

Unter "C₁₋₁₂-Alkylgruppe" wird im Sinne der vorliegenden Erfindung ein verzweigter oder geradkettiger Alkylrest 1 bis 12 Kohlenstoffatomen verstanden. Als Beispiele seien eine Methyl-, Ethyl-, n-Propyl-, i-Propyl-, n-Butyl-, i-Butyl-, tert.-Butyl-, n-Pentyl-, i-Pentyl-, n-Hexyl-, 2-Methylpentyl-, 3-Methylpentyl-, 2,2-Dimethylbutyl-, 2,3-Dimethylbutylgruppe, eine Octyl-, Nonyl-, Decyl- oder Undecylgruppe genannt.

Die vorstehend genannte "C₃₋₈-Cycloalkylgruppe" bedeutet erfindungsgemäß eine mono- oder bicyclische Gruppe, wie beispielsweise eine Cyclopropyl-, Cyclobutyl-, Cyclopentyl- oder Cyclohexylgruppe.

Unter dem Begriff "Arylgruppe" wird im Sinne der vorliegenden Anmeldung ein substituierter oder unsubstituierter Arylrest mit 6 bis 15 Kohlenstoffatomen, beispielsweise eine Phenylgruppe, eine substituierte Phenylgruppe, wie eine Halogenphenylgruppe oder eine Nitrophenylgruppe, oder eine Naphtylgruppe verstanden.

Unter dem Begriff "Aryl-C₁₋₄-Alkylgruppe" wird im Sinne der vorliegenden Anmeldung ein mit einem Arylrest substituierter Alkylrest, die zusammen 7 bis 15 Kohlenstoffatomen aufweisen, verstanden, wobei der Arylrest weitere Substituenten, wie beispielsweise ein Halogenatom tragen kann. Beispiele sind eine freie oder aromatisch substituierte Benzylgruppe, wie eine Benzylgruppe oder eine Halogenbenzylgruppe.

Unter dem Begriff "Halogenatom" wird im Rahmen der vorliegenden Erfindung ein Fluor-, Chlor-, Brom- oder Iodatom verstanden.

Der Rest der Formel CnF2n+1, wobei n=1, 2, 3, 4, 5 oder 6, kann ein verzweigter oder geradkettiger Fluoralkylrest mit 1 bis 6 Kohlenstoffatomen sein, wobei Beispiele eine Trifluormethyl-, Pentafluorethyl-, Heptafluor-n-propyl- oder Heptafluor-iso-propylgruppe sind, wobei eine Trifluormethyl- oder Pentafluorethylgruppe erfindungsgemäß bevorzugt sind.

Wenn STEROID für ein steroidales Ringsystem der Teilformel A steht, bedeutet R₇ vorzugsweise ein Wasserstoffatom, ein Chloratom oder eine Hydroxygruppe, R¹⁰ vorzugsweise ein Wasserstoffatom oder eine Hydroxygruppe und R⁹ vorzugsweise ein Wasserstoffatom oder ein Fluoratom.

Wenn STEROID für ein steroidales Ringsystem der Teilformel B steht, bedeutet R₇ vorzugsweise ein Wasserstoffatom, ein Chloratom oder eine Hydroxygruppe und R₈ vorzugsweise ein Wasserstoffatom oder eine Methylgruppe.

Wenn STEROID für ein steroidales Ringsystem der Teilformel C steht, bedeutet R₇ vorzugsweise ein Wasserstoffatom, ein Chloratom oder eine Hydroxygruppe und R¹¹ vorzugsweise eine Hydroxymethylengruppe oder eine Formylgruppe oder R¹¹ und R¹² stehen zusammen für eine Doppelbindung.

Wenn STEROID für ein steroidales Ringsystem der Teilformel D steht, bedeutet R₇ vorzugsweise ein Wasserstoffatom, ein Chloratom oder eine Hydroxygruppe, R₈ vorzugsweise ein Wasserstoffatom oder eine Methylgruppe und R¹³ und R₇ bedeuten vorzugsweise zusammen eine Doppelbindung und Y bedeutet vorzugsweise ein Sauerstoffatom.

Wenn STEROID für ein steroidales Ringsystem der Teilformel E steht, bedeutet R₈ vorzugsweise ein Wasserstoffatom oder eine Methylgruppe und R¹⁴ und R¹⁵ stehen vorzugsweise zusammen für einen Thiiranring oder einen [3,2c]Pyrazolring,

Wenn STEROID für ein steroidales Ringsystem der Teilformel F steht, bedeutet R¹¹ vorzugsweise eine C₁₋₄-Alkylgruppe oder Nitrilgruppe .

R¹ bedeutet vorzugsweise eine Methylgruppe.

R² bedeutet vorzugsweise eine Hydroxygruppe, eine Formyloxygruppe, Acetyloxygruppe, Propionyloxygruppe, Butyryloxygruppe, [(trans-4-Butylcyclohexyl)carbonyl]oxygruppe, Phenylpropionyloxygruppe, iso-Butyryloxygruppe, Heptanyloxygruppe oder Undecanyloxygruppe.

R³ bedeutet vorzugsweise eine Trifluormethylgruppe oder eine Pentafluorethylgruppe.

Besonders bevorzugte 17α-Fluoralkylsteroide sind nachfolgend aufgeführt:
1) 17β-Hydroxy-17α-trifluormethyl-7α-methyl-androst-4-en-3-on,
2) 17β,4-Dihydroxy-17α-trifluormethyl-androst-4-en-3-on,
3) 17β-Hydroxy-17α-trifluormethyl-4-chlor-androst-4-en-3-on,
4) 17β-Hydroxy-17α-trifluormethyl-4-brom-androst-4-en-3-on,
5) 17β-Hydroxy-17α,4-bis(trifluormethyl)-androst-4-en-3-on,
6) 17β, 11β-Dihydroxy-17α-trifluormethyl-androst-4-en-3-on,
7) 17β,11β-Dihydroxy-17α-trifluormethyl-9α-fluor-androst-4-en-3-on,
8) 17β-Hydroxy-17α-trifluormethyl-androst-1,4-dien-3-on,
9) 17β-Hydroxy-17α-trifluormethyl-4-chlor-androst-1,4-dien-3-on,
10) 17β,4-Dihydroxy-17α-trifluormethyl-androst-1,4-dien-3-on,
11) 17β-Hydroxy-17α-trifluormethyl-7α-methyl-androst-1,4-dien-3-on,
12) 17β-Hydroxy-17α-trifluormethyl-7α-methyl-4-chlor-androst-1,4-dien-3-on,
13) 17β-Hydroxy-17α-pentafluorethyl-androst-4-en-3-on,
14) 17β-Hydroxy-17α-pentafluorethyl-7α-methyl-androst-4-en-3-on,
15) 17β,4-Dihydroxy-17α-pentafluorethyl-androst-4-en-3-on,
16) 17β-Hydroxy-17α-pentafluorethyl-4-chlor-androst-4-en-3-on,
17) 17β-Hydroxy-17α-pentafluorethyl-4-brom-androst-4-en-3-on,
18) 17β-Hydroxy-17α-pentafluorethyl-4-trifluormethyl-androst-4-en-3-on,
19) 17β, 11β-Dihydroxy-17α-pentafluorethyl-androst-4-en-3-on,
20) 17β, 11β-Dihydroxy-17α-pentafluorethyl-9α-fluor-androst-4-en-3-on,
21) 17β-Hydroxy-17α-pentafluorethyl-androst-1,4-dien-3-on,
22) 17β-Hydroxy-17α-pentafluorethyl-4-chlor-androst-1,4-dien-3-on,
23) 17β,4-Dihydroxy-17α-pentafluorethyl-androst-1,4-dien-3-on,
24) 17β-Hydroxy-17α-pentafluorethyl-4-trifluormethyl-androst-1,4-dien-3-on,
25) 17β-Hydroxy-17α-pentafluorethyl-7α-methyl-androst-1,4-dien-3-on,
26) 17β-Hydroxy-17α-pentafluorethyl-7α-methyl-4-chlor-androst-1,4-dien-3-on,
27) 17β-Hydroxy-17α-trifluormethyl-7α-methyl-estr-4-en-3-on,
28) 17β,4-Dihydroxy-17α-trifluormethyl-estr-4-en-3-on,
29) 17β-Hydroxy-17α-trifluormethyl-4-chlor-estr-4-en-3-on,
30) 17β-Hydroxy-17α-trifluormethyl-4-brom-estr-4-en-3-on,
31) 17β-Hydroxy-17α,4-bis(trifluormethyl)-estr-4-en-3-on,
32) 17β-Hydroxy-17α-trifluormethyl-7α-methyl-estr-4,9-dien-3-on,
33) 17β-Hydroxy-17α-trifluormethyl-7α-methyl-estr-4,9,11-trien-3-on,
34) 17β-Hydroxy-17α-pentafluorethyl-estr-4-en-3-on,
35) 17β-Hydroxy-17α-pentafluorethyl-7α-methyl-estr-4-en-3-on,
36) 17β,4-Dihydroxy-17α-pentafluorethyl-estr-4-en-3-on,
37) 17β-Hydroxy-17α-pentafluorethyl-4-chlor-estr-4-en-3-on,
38) 17β-Hydroxy-17α-pentafluorethyl-4-brom-estr-4-en-3-on,
39) 17β-Hydroxy-17α-pentafluorethyl-4-trifluormethyl-estr-4-en-3-on,
40) 17β-Hydroxy-17α-pentafluorethyl-estr-4,9-dien-3-on,
41) 17β-Hydroxy-17α-pentafluorethyl-estr-4,9,11-trien-3-on,
42) 17β-Hydroxy-17α-pentafluorethyl-7α-methyl-estr-4,9-dien-3-on,
43) 17β-Hydroxy-17α-pentafluorethyl-7α-methyl-estr-4,9,11-trien-3-on,
44) 13-Ethyl-17β-hydroxy-17α-trifluormethyl-4-chlor-gon-4-en-3-on,
45) 13-Ethyl-17β,4-dihydroxy-17α-trifluormethyl-gon-4-en-3-on,
46) 13-Ethyl-17β-hydroxy-17α-trifluormethyl-7α-methyl-gon-4-en-3-on,
47) 13-Ethyl-17β-hydroxy-17α-trifluormethyl-7α-methyl-gon-4,9-dien-3-on,
48) 13-Ethyl-17β-hydroxy-17α-trifluormethyl-7α-methyl-gon-4,9,11-trien-3-on,
49) 13-Ethyl-17β-hydroxy-17α-pentafluorethyl-gon-4-en-3-on,
50) 13-Ethyl-17β-hydroxy-17α-pentafluorethyl-7α-methyl-gon-4-en-3-on,
51) 13-Ethyl17β,4-Dihydroxy-17α-pentafluorethyl-gon-4-en-3-on,
52) 13-Ethyl-17β-hydroxy-17α-pentafluorethyl-4-chlor-gon-4-en-3-on,
53) 13-Ethyl-17β-hydroxy-17α-pentafluorethyl-4-brom-gon-4-en-3-on,
54) 13-Ethyl-17β-hydroxy-17α-pentafluorethyl-4-trifluormethyl-gon-4-en-3-on,
55) 13-Ethyl-17β-hydroxy-17α-pentafluorethyl-gon-4,9-dien-3-on,
56) 13-Ethyl-17β-hydroxy-17α-pentafluorethyl-gon-4,9, 11-trien-3-on,
57) 13-Ethyl-17β-hydroxy-17α-pentafluorethyl-7α-methyl-gon-4,9-dien-3-on,
58) 13-Ethyl-17β-hydroxy-17α-pentafluorethyl-7α-methyl-gon-4,9, 11-trien-3-on,
59) 17β-Hydroxy-17α-trifluormethyl-5α-androstan-3-on,
60) 17β-Hydroxy-17α-pentafluorethyl-5α-androstan-3-on,
61) 17β-Hydroxy-17α-trifluormethyl-7α-methyl-5α-androstan-3-on,
62) 17β-Hydroxy-17α-pentafluorethyl-7α-methyl-5α-androstan-3-on,
63) 17β-Hydroxy-17α-trifluormethyl-2-hydroxymethylen-5α-androstan-3-on,
64) 17β-Hydroxy-17α-pentafluorethyl-2-hydroxymethylen-5α-androstan-3-on,
65) 17β-Hydroxy-17α-trifluormethyl-2α-methyl-5α-androstan-3-on,
66) 17β-Hydroxy-17α-pentafluorethyl-2α-methyl-5α-androstan-3-on,
67) 17β-Hydroxy-17α-trifluormethyl-1α-methyl-5α-androstan-3-on,
68) 17β-Hydroxy-17α-pentafluorethyl-1α-methyl-5α-androstan-3-on,
69) 17β-Hydroxy-17α-trifluormethyl-5α-androst-2-en,
70) 17β-Hydroxy-17α-pentafluorethyl-5α-androst-2-en,
71) 17β-Hydroxy-17α-trifluormethyl-2-methyl-5α-androst-2-en,
72) 17β-Hydroxy-17α-pentafluorethyl-2-methyl-5α-androst-2-en,
73) 17β-Hydroxy-17α-trifluormethyl-2-cyano-5α-androst-2-en,
74) 17β-Hydroxy-17α-pentafluorethyl-2-cyano-5α-androst-2-en,
75) 17β-Hydroxy-17α-trifluormethyl-2-formyl-5α-androst-2-en,
76) 17β-Hydroxy-17α-pentafluorethyl-2-formyl-5α-androst-2-en,
77) 17β-Hydroxy-17α-trifluormethyl-[2,3c]oxadiazol-5α-androstan,
78) 17β-Hydroxy-17α-pentafluorethyl-[2,3c]oxadiazol-5α-androstan,
79) 17β-Hydroxy-17α-trifluormethyl-[3,2c]isoxazol-5a-androstan,
80) 17β-Hydroxy-17α-pentafluorethyl-[3,2c]isoxazol-5α-androstan,
81) 17β-Hydroxy-17α-trifluormethyl-[3,2c]pyrazol-5α-androstan,
82) 17β-Hydroxy-17α-pentafluorethyl-[3,2c]pyrazol-5α-androstan,
83) 17β-Hydroxy-17α-trifluormethyl-2β,3β-epithio-5α-androstan,
84) 17β-Hydroxy-17α-pentafluorethyl-2β,3β-epithio-5α-androstan,
85) 17β-Hydroxy-17α-trifluormethyl-2α,3α-epithio-5α-androstan,
86) 17β-Hydroxy-17α-pentafluorethyl-2α,3α-epithio-5α-androstan,
87) 17β-Hydroxy-17α-trifluormethyl-2-oxa-5α-androstan-3-on,
88) 17β-Hydroxy-17α-pentafluorethyl-2-oxa-5α-androstan-3-on,
89) 17β-Hydroxy-17α-trifluormethyl-5α-androst-1-en-3-on,
90) 17β-Hydroxy-17α-pentafluorethyl-5α-androst-1-en-3-on,
91) 17β-Hydroxy-17α-trifluormethyl-1-methyl-5α-androst-1-en-3-on,
92) 17β-Hydroxy-17α-pentafluorethyl-1-methyl-5α-androst-1-en-3-on,
93) 17β-Hydroxy-17α-trifluormethyl-2-methyl-5α-androst-1-en-3-on und
94) 17β-Hydroxy-17α-pentafluorethyl-2-methyl-5α-androst-1-en-3-on.

Ein weiterer Gegenstand der vorliegeneden Erfindung ist das Verfahren zur Herstellung von 17α-Fluoralkylsteroiden der allgemeinen Formel (I), in dem Verbindungen der allgemeinen Formel (II) 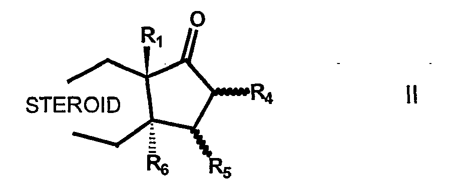 in der R¹, R⁴, R⁵, R⁶ und STEROID, die in Anspruch 1 gegebene Bedeutung haben, in Gegenwart von Fluorid mit Perfluoralkyltrialkylsilanen, (Alk)₃SiCnF2n+1, oder mit Fluoralkyllithium, LiCnF2n+1, bzw. Fluoralkylgrignardreagenzien, ZMgCnF2n+1,
umgesetzt werden, wobei n = 1, 2, 3, 4, 5 oder 6, m > 1 und m+o = 2n ist, Z ein Chlor-, Brom- oder lodatom ist und Alk ein C₁₋₄-Alkylrest ist.

Die Einführung der fluorierten 17α-Alkylkette kann durch Addition von (Perfluoralkyl)trimethylsilanen in Gegenwart von Fluorid (Rupperts Reagenz und seine Homologen , J. Org. Chem. 1991, 56, 984-989) oder durch Addition von Fluoralkyllithium bzw. Fluoralkylgrignardreagenzien an die 17-Oxo-Gruppe der allgemeinen Formel II erfolgen. Die Einführung von 17α-Perfluoralkylketten kann durch Addition von Perfluoralkyllithium an die 17-Oxo-Gruppe der allgemeinen Formel II erfolgen (Tetr. Lett. 1985, 26, 5243; J. Org. Chem. 1987, 52, 2481).
Die in der allgemeinen Formel II genannten Substituenten R⁴, R⁵ und R⁶ werden in den Steroid-D-Ring günstigerweise vor der Einführung der fluorierten 17α-Alkylkette, nach dem Fachmann bekannten Methoden, eingeführt.
Zur Herstellung der Verbindungen der allgemeinen Formel II mit den Teilstrukturen A bis F kann man sich bekannter Steroidgrundkörper bedienen.
Folgende Steroidgrundkörper können Beispielsweise verwendet werden:
Für den Steroidgrundkörper A: Androst-4-en-3,17-dion und Dehydroepiandrosteron.
Für den Steroidgrundkörper B: 19-Nortestosteron und 3,3-Dimethoxy-estr-5(10)-17-on (DD 79-213049).
Für die Steroidgrundkörper C, D bzw. E : Epiandrosteron.
Für den Steroidgrundkörper F: 5α-Androst-2-en-17-on aus Epiandrosteron (US-A-3,098,851).
Die in den Teilstrukturen der Ausgangsmaterialien für die Steroidgrundkörper A bis F enthaltenen funktionellen Gruppen können gegebenenfalls nach dem Fachmann bekannten Methoden geschützt werden.
So können Ketogruppen in den Ausgangsmaterialien der Teilstrukturen A bis F als Ketale oder Thioacetale nach dem Fachmann bekannten Methoden geschützt werden.
Die Einführung der Substituenten R⁷ bis R¹⁵ in die Teilstrukturen A bis F kann sowohl vor als auch nach dem Einbau der fluorierten 17α-Alkylkette nach dem Fachmann bekannten Methoden erfolgen.

Die erfindungsgemäßen Verbindungen besitzen androgene Aktivität, wie nachfolgende Rezeptorbindungsaffinitäten an den Androgenrezeptor illustrieren.

| Verbindung | RBA % |
|---|---|
| Testosteron | 35 |
| R 1881 (Methyltrienolon) | 100 |
| 17β,4-Dihydroxy-17α-trifluormethyl-estr-4-en-3-on | 15 |
| 17β-Hydroxy-17α-trifluormethyl-7α-methyl-estr-4-en-3-on | 40 |
| 17β-Hydroxy-17α-trifluormethyl-4-chlor-estr-4-en-3-on | 41 |
| 17β-Hydroxy-17α-trifluormethyl-androst-4-en-3-on Vergleich beispiel | 9,4 |
| 17β-Hydroxy-17α-trifluormethyl-4-chlor-androst-4-en-3-on | 20 |
| 17β,11 β-Dihydroxy-17α-trifluormethyl-androst-4-en-3-on | 8,6 |
| 17β-Hydroxy-17α-trifluormethyl-7α-methyl-androst-4-en-3-on | 30 |
| 17β-Hydroxy-17α-pentafluorethyl-estr-4-en-3-on | 21 |
| 17β-Hydroxy-17α-pentafluorethyl-7α-methyl-estr-4-en-3-on | 34 |
| 17β,4-Dihydroxy-17α-pentafluorethyl-estr-4-en-3-on | 20 |
| 17β-Hydroxy-17α-pentafluorethyl-4-chlor-estr-4-en-3-on | 27 |

Diese Testergebnisse eröffnen den erfindungsgemäßen Verbindungen der allgemeinen Formel (I) vielfältige Möglichkeiten für die Fertilitätskontrolle beim Mann, die Hormon-Replacement-Therapie (HRT) bei Mann und Frau oder die Behandlung hormonell bedingter Erkrankungen bei Mann und Frau, wie beispielsweise Endometriose, Mammacarcinom oder Hypogonadismus.

Gegenstand der vorliegenden Erfindung sind deshalb auch pharmazeutische Zusammensetzungen, die mindestens ein 17α-Fluoralkylsteroid der allgemeinen Formel (I), gegebenenfalls zusammen mit pharmazeutisch verträglichen Hilfs- und Trägerstoffen enthalten.

Diese pharmazeutischen Zusammensetzungen und Arzneimittel können zur oralen, rektalen, vaginalen, subcutanen, percutanen, intravenösen oder intramuskulären Applikation vorgesehen sein. Sie enthalten neben üblichen Träger- und/oder Verdünnungsmitteln mindestens eine Verbindung der allgemeinen Formel I.

Die Arzneimittel der Erfindung werden mit den üblichen festen oder flüssigen Trägerstoffen oder Verdünnungsmitteln und den üblicherweise verwendeten pharmazeutisch-technischen Hilfsstoffen entsprechend der gewünschten Applikationsart mit einer geeigneten Dosierung in bekannter Weise hergestellt. Die bevorzugten Zubereitungen bestehen in einer Darreichungsform, die zur oralen Applikation geeignet ist. Solche Darreichungsformen sind beispielsweise Tabletten, Filmtabletten, Dragees, Kapseln, Pillen, Pulver, Lösungen oder Suspensionen oder auch Depotformen.

Selbstverständlich kommen auch parenterale Zubereitungen wie Injektionslösungen in Betracht. Weiterhin seien als Zubereitungen beispielsweise auch Suppositorien und Mittel zur vaginalen Anwendung genannt.

Entsprechende Tabletten können beispielsweise durch Mischen des Wirkstoffs mit bekannten Hilfsstoffen, beispielsweise inerten Verdünnungsmitteln wie Dextrose, Zucker, Sorbit, Mannit, Polyvinylpyrrolidon, Sprengmitteln wie Maisstärke oder Alginsäure, Bindemitteln wie Stärke oder Gelantine, Gleitmitteln wie Magnesiumstearat oder Talk und/oder Mitteln zur Erzielung eines Depoteffektes wie Carboxylpolymethylen, Carboxylmethylcellulose, Celluloseacetatphthalat oder Polyvinylacetat, erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Polyvinylpyrrolidon oder Schellack, Gummitarabicum, Talk, Titanoxid oder Zucker, hergestellt werden. Dabei kann auch die Drageehülle aus mehreren Schichten bestehen, wobei die oben bei den Tabletten erwähnten Hilfsstoffe verwendet werden können.

Lösungen oder Suspensionen mit den erfindungsgemäßen Verbindungen der allgemeinen Formel I können zusätzlich geschmacksverbessernde Mittel wie Saccharin, Cyclamat oder Zucker sowie z. B. Aromastoffe wie Vanillin oder Orangenextrakt enthalten. Sie können außerdem Suspendierhilfsstoffe wie Natriumcarboxymethylcellulose oder Konservierungsstoffe wie p-Hydroxybenzoate enthalten.

Die Verbindungen der allgemeinen Formel I enthaltende Kapseln können beispielsweise hergestellt werden, indem man die Verbindung(en) der allgemeinen Formel I mit einem inerten Träger wie Milchzucker oder Sorbit mischt und in Gelatinekapseln einkapselt.

Geeignete Suppositorien lassen sich beispielsweise durch Vermischen mit dafür vorgesehenen Trägermitteln wie Neutralfetten oder Polyethylenglykol bzw. deren Derivaten herstellen.

Die nachfolgenden Beispiele erläutern die vorliegende Erfindung, ohne sie einzuschränken.

### Vergleichsbeispiel 1

### 17β-Hydroxy-17α-trifluormethyl-androst-4-en-3-on

10 g 3β-Acetoxy-Dehydroepiandrosteron werden in 300 ml THF gelöst und mit 0,5 g Tetrabutylammoniumfluorid versetzt. Unter Rühren bei Raumtemperatur werden langsam 15 ml Trifluormethyltrimethylsilan zugetropft und es wird 3 Stunden gerührt. Dann gibt man 200 ml halbkonzentrierte Natriumhydrogencarbonatlösung zu und destilliert das THF im Vakuum ab. Der Rückstand wird drei mal mit 100 ml Essigsäureethylester extrahiert. Die vereinigten organischen Extrakte werden getrocknet, eingeengt und an Kieselgel chromatographiert. Man erhält 12 g 3β-Acetoxy-17β-trimethylsilyloxy-17α-trifluormethyl-androst-5-en.
12 g 3β-Acetoxy-17β-trimethylsilyloxy-17α-trifluormethyl-androst-5-en werden in 100 ml THF gelöst und bei Raumtemperatur mit 20 ml 30 %-iger Fluorwasserstoffsäure versetzt. Nach 3 Stunden gießt man auf 200 ml 12%-ige Ammoniaklösung, extrahiert mit 3 mal 100 ml Essigester, trocknet die organischen Extrakte und engt ein. Man erhält 9 g 3β-Acetoxy-17β-hydroxy-17α-trifluormethyl-androst-5-en welches in 300 ml Methanol gelöst und mit 6 g Kaliumhydroxid versetzt wird. Nach 30 Minuten Rühren bei Raumtemperatur wird mit 2n Salzsäure neutralisiert und das Methanol wird im Vakuum abgezogen. Der Rückstand wird mit 4 mal 100 ml Essigsäureethylester ausextrahiert und die vereinigten organischen Extrakte werden getrocknet und eingeengt. Man erhält 7,5 g 3β,17β-Dihydroxy-17α-trifluormethyl-androst-5-en, welches mit 80 ml Cyclohexanon, 5 g Aluminiumtriisopropanolat und 250 ml Toluol 3 Stunden am Rückfluß erhitzt wird. Man läßt abkühlen, versetzt mit 200 ml 2n Natrium-Kalium-Tartrat-Lösung, trennt die organische Phase ab und extrahiert mit 2 mal 100 ml Essigsäureethylester nach. Die vereinigten organischen Extrakte werden eingeengt, der Rückstand wird chromatografisch gereinigt und aus Methanol kristallisiert. Man erhält 17β-Hydroxy-17α-trifluormethyl-androst-4-en-3-on.
¹H-NMR (DMSO-D6): 0,89 (s, 3H, H-18), 1,15 (s, 3H, H-19), 5,62 (s, 1H, H-4) ¹⁹F-NMR: -75.3

### Beispiel 2

### 17β-Hydroxy-17α-trifluormethyl-7α-methyl-androst-4-en-3-on

7 g 17β-Hydroxy-17α-trifluormethyl-androst-4-en-3-on werden mit 8,5 g Chloranil in 200 ml tert.-Butanol 30 Minuten am Rückfluß gekocht. Man läßt abkühlen und engt zur Trockene ein. Der Rückstand wird an Kieselgel chromatographiert. Zur weiteren Reinigung wird aus Dichlormethan/Hexan umkristallisiert. Man erhält 17β-Hydroxy-17α-trifluormethyl-androst-4,6-dien-3-on.
¹H-NMR: 1,04 (s, 3H, H-18), 1,13 (s, 3H, H-19), 5,69 (s, 1 H, H-4), 6,11 (m, 2H, H-6, H-7)
¹⁹F-NMR : -75.3

Zu einer Lösung von Methylmagesiumiodid (bereitet aus 2,5 g Magnesium und 6,4 ml Methyliodid in 80 ml Diethylether) werden 80 ml THF gegeben , man kühlt auf -5°C und gibt 1 g Kupferacetat-Monohydrat, gelöst in 50 ml THF, zu. Es wird auf -20°C gekühlt, dann wird eine Lösung von 5 g 17β-Hydroxy-17α-trifluormethyl-androst-4,6-dien-3-on zugegeben in 80 ml THF zugetropft. Nach 2 Stunden gießt man auf Eiswasser/2N Schwefelsäure und extrahiert mit 3 mal 80 ml Essigester. Der organische Extrakt wird getrocknet und eingeengt. Der Rückstand wird an Kieselgel chromatographiert. Zur weiteren Reinigung wird aus Essigester umkristallisiert. Man erhält 17β-Hydroxy-17α-trifluormethyl-7α-methyl-androst-4-en-3-on.
¹H-NMR: 0,77 (d, J=7Hz, 3H, H-7-Methyl), 0,99 (s, 3H, H-18), 1.20 (s, 3H, H-19), 5.73 (m, 1H, H-4)
¹⁹F-NMR: -75.3

### Beispiel 3

### 17β-Hydroxy-17α-trifluormethyl-4-chlor-androst-4-en-3-on

### Stufe 1

### 17β-Hydroxy-17α-trifluormethyl-4ξ, 5ξ-epoxy-androstan-3-on

2g 17β-Hydroxy-17α-trifluormethyl-androst-4-en-3-on werden in 120 ml Methanol sowie 70 ml THF gelöst und bei 10°C mit 20 ml Wasserstoffperoxidlösung (35%) versetzt. Unter Rühren werden 5 ml 10 %-ige Natriumhydroxidlösung zugegeben und es wird 3 Stunden gerührt. Die Reaktionslösung wird auf 50 ml eingeengt, dann mit 50 ml Dichlormethan und 25 ml Wasser versetzt und die organische Phase wird abgetrennt. Man wäscht mit halbkonzentrierter Thiosulfatlösung, trocknet und engt bis zur Trockene ein. Der erhaltene Rückstand besteht aus einem Gemisch von 4α,5α- bzw. 4β,5β-Epoxiden und wird ohne weitere Reinigung in der Folgestufe eingesetzt.

### Stufe 2

### 17β-Hydroxy-17α-trifluormethyl-4-chlor-androst-4-en-3-on

2 g Epoxidgemisch (Stufe 1) werden in 200 ml Aceton gelöst und bei 5°C mit 12 ml konzentrierter Salzsäure versetzt. Nach 2 Stunden wird mit Sodalösung neutralisiert und das Aceton wird abgezogen. Der Rückstand wird mit Dichlormethan ausextrahiert. Die organischen Extrakte werden getrocknet und eingeengt. Nach Kristallisation aus Dichlormethan/Hexan erhält man 17β-Hydroxy-17α-trifluormethyl-4-chlor-androst-4-en-3-on.
¹H-NMR: 0,99 (s, 3H, H-18), 1,24 (s, 3H, H-19)
¹⁹F-NMR: -75,3

### Beispiel 4

### 17β,4-Dihydroxy-17α-trifluormethyl-androst-4-en-3-on

2 g Epoxidgemisch (Stufe 1, Herstellung 17β-Hydroxy-17α-trifluormethyl-4-chlor-androst-4-en-3-on) werden in 20 ml Essigsäure, welche 2 Vol.-% konzentrierte Schwefelsäure enthält, gelöst. Die Lösung wird 24 h bei 10 °C stehengelassen. Danach versetzt man mit 200 ml Essigsäureethylester und neutralisiert mit Sodalösung. Die organische Phase wird getrocknet und eingeengt. Der Rückstand wird an Kieselgel chromatographiert und aus Essigsäureethylester/Hexan kristallisiert.
¹H-NMR: 0,99 (s, 3H, H-18), 1,19 (s, 3H, H-19), 6,10 (s, 1H, 4-OH)
¹⁹F-NMR : -75,3

### Beispiel 5

### 17β-Hydroxy-17α-trifluormethyl-androst-1,4-dien-3-on

2 g 17β-Hydroxy-17α-trifluormethyl-androst-4-en-3-on werden mit 1,8 g DDQ in 60 ml Toluol 60 Stunden bei 85 °C gerührt. Es wird vom Niederschlag abfiltriert, mit Toluol nachgewaschen und das Filtrat wird eingeengt. Der Rückstand wird an Kieselgel chromatographiert und aus Essigsäureethylester umkristallisiert.
¹H-NMR: 1,02 (s, 3H, H-18), 1,24 (s, 3H, H-19), 6,07 (m, 1 H, H-4), 6,22 (dd, J =1,6, 10 Hz, 1 H, H-2), 7,04 (d, J = 10 Hz, 1 H, H-1)
¹⁹F-NMR : -75,4

### Beispiel 6

### 17β-Hydroxy-17α-trifluormethyl-4-chlor-androst-1,4-dien-3-on

Herstellung aus 17β-Hydroxy-17α-trifluormethyl-4-chlor-androst-4-en-3-on analog der Vorschrift für 17β-Hydroxy-17α-trifluormethyl-androst-1,4-dien-3-on.
¹H-NMR: 1,02 (s, 3H, H-18), 1,31 (s, 3H, H-19), 6,36 (d,J=10 Hz, 1H, H-2), 7.07 (d, J = 10 Hz, 1H, H-1)
¹⁹F-NMR : -75,8

### Beispiel 7

### 17β-Hydroxy-17α-trifluormethyl-7α-methyl-estr-4-en-3-on

### Stufe 1

### 7α-Methyl-estr-4-en-3,17-dion

10 g 17β-Hydroxy-7α-methyl-estr-4-en-3-on (Herstellung: Steroids 1963, 317) werden in 200 ml Aceton gelöst und bei -20 °C mit 15 ml 8n Chromschwefelsäure oxidiert. Nach beendeter Reaktion werden 10 ml Methanol zugegeben und man läßt auf Raumtemperatur erwärmen. Die Lösungsmittel werden im Vakuum abgezogen und der Rückstand wird mit 300 ml Wasser versetzt, wobei das Produkt ausfällt. Es wird abgesaugt und man erhält 6,5 g 7α-Methyl-estr-4-en-3,17-dion.

### Stufe 2

### 3,3-Ethylendithio-7α-methyl-estr-4-en-17-on

5 g 7α-Methyl-estr-4-en-3,17-dion werden in 50 ml Methanol gelöst und mit 3 ml Ethandithiol versetzt. Man gibt 1,5 ml Bortrifluorid-Diethyletherat zu und rührt bei Raumtemperatur 2 Stunden wobei das Produkt auskristallisiert. Es wird abgesaugt und man erhält 6 g 3,3-Ethylendithio-7α-methyl-estr-4-en-17-on.

### Stufe 3

### 17β-Hydroxy-17α-trifluormethyl-7α-methyl-estr-4-en-3-on

5 g 3,3-Ethylendithio-7α-methyl-estr-4-en-17-on werden bei Raumtemperatur in 150 ml THF mit 0,25 g Tetrabutylammoniumfluorid versetzt und man tropft langsam 8 ml Trifluormethyltrimethylsilan zu. Nach 3 Stunden Rühren gibt man 200 ml halbkonzentrierte Natriumhydrogencarbonatlösung zu und destilliert das THF im Vakuum ab. Der Rückstand wird dreimal mit 100 ml Essigsäureethylester extrahiert. Die vereinigten organischen Extrakte werden getrocknet, eingeengt und an Kieselgel chromatographiert. Man erhält 3 g 17β-Trimethylsilyloxy-17α-trifluormethyl-3,3-ethylendithio-7α-methyl-estr-4-en.

3 g 17β-Trimethylsilyloxy-17α-trifluormethyl-3,3-ethylendithio-7α-methyl-estr-4-en werden in 40 ml THF gelöst und bei Raumtemperatur mit 5 ml 30 %-iger Fluorwasserstoffsäure versetzt. Nach 3 Stunden gießt man auf 200 ml 12 %-ige Ammoniaklösung, extrahiert mit 3 mal 100 ml Essigester, trocknet die organischen Extrakte und engt ein. Der Rückstand wird in 100 ml 95 %-igem Methanol aufgenommen, mit 9 ml Methyliodid sowie 2,5 g Calciumcarbonat versetzt und 20 Stunden am Rückfluß erhitzt. Nach dem Abkühlen wird abgesaugt und das Filtrat wird zur Trockene eingeengt. Der Rückstand wird an Kieselgel chromatographiert und aus Dichlormethan/Hexan kristallisiert. Man erhält 17β-Hydroxy-17α-trifluormethyl-7α-methyl-estr-4-en-3-on.
¹H-NMR (D6-DMSO): 0,70 (d, J = 7,7 Hz, 3H, 7-Methyl), 0,93 (s, 3H, H-18), 5,71 (s, 1H, H-4)
¹⁹F-NMR : -75,5

### Beispiel 8

### 17β-Hydroxy-17α-trifluormethyl-4-chlor-estr-4-en-3-on und 17β,4-Dihydroxy-17α-trifluormethyl -estr-4-en-3-on

### Stufe 1

### 17β-Hydroxy-17α-trifluormethyl-estr-4-en-3-on

10 g 3,3-Dimethoxy-estr-5(10)-en-17-on werden in 300 ml THF gelöst und mit 0,5 g Tetrabutylammoniumfluorid versetzt. Unter Rühren bei Raumtemperatur werden langsam 15 ml Trifluormethyltrimethylsilan zugetropft und es wird 3 Stunden gerührt. Dann gibt man 200 ml halbkonzentrierte Natriumhydrogencarbonatlösung zu und destilliert das THF im Vakuum ab. Der Rückstand wird dreimal mit 100 ml Essigsäureethylester extrahiert. Die vereinigten organischen Extrakte werden getrocknet und eingeengt. Man erhält 17β-Trimethylsilyloxy-17α-trifluormethyl-3,3-dimethoxy-estr-5(10)-en.
12 g 17β-Trimethylsilyloxy-17α-trifluormethyl-3,3-dimethoxy-estr-5(10)-en werden in 100 ml THF gelöst und bei Raumtemperatur mit 20 ml 30 %-iger Fluorwasserstoffsäure versetzt. Nach 24 Stunden gießt man auf 200 ml 12 %-ige Ammoniaklösung, extrahiert mit 3 mal 100 ml Essigester, trocknet die organischen Extrakte und engt ein. Der Rückstand wird durch Chromatographie an Kieselgel gereinigt und man erhält 17β-Hydroxy-17α-trifluormethyl-estr-4-en-3-on.
¹H-NMR(CDCl₃): 1,00 (s, 3H, H-18), 5,82 (s, 1H, H-4)
¹⁹F-NMR : -75,1

### Stufe 2

### 17β-Hydroxy-17α-trifluormethyl-4ξ,5ξ-Epoxy -estran-3-on

Die Herstellung erfolgt analog 17β-Hydroxy-17α-trifluormethyl-4ξ,5ξ-epoxy-androstan-3-on. Der erhaltene Rückstand besteht aus einem Gemisch von 4α,5α- bzw. 4β,5β-Epoxiden und wird ohne weitere Reinigung in der Folgestufe eingesetzt.

### Stufe 3

### 17β-Hydroxy-17α-trifluormethyl-4-chlor-estr-4-en-3-on

Die Herstellung erfolgt aus 17β-Hydroxy-17α-trifluormethyl-4ξ, 5ξ-epoxy-estran-3-on analog zu 17β-Hydroxy-17α-trifluormethyl-4-chlor-androst-4-en-3-on.
¹H-NMRv(CDCl₃): 1,00 (s, 3H, H-18)
¹⁹F-NMR : -75,3

### Stufe 4

### 17β,4-Dihydroxy-17α-trifluormethyl-estr-4-en-3-on

Die Herstellung erfolgt aus 17β-Hydroxy-17α-trifluormethyl-4ξ, 5ξ-epoxy-estran-3-on analog zu 17β,4-Dihydroxy-17α-trifluormethyl -androst-4-en-3-on.
¹H-NMR (CDCl₃): 1,00 (s,3H,H-18), 6,1 (s, 1H, 4-OH)
¹⁹F-NMR : -75,3

### Beispiel 9

### 17β-Hydroxy-17α-pentafluorethyl -androst-4-en-3-on

20 g 3,3-Ethylendithio-androst-4-en-17-on werden in 600 ml Diethylether suspendiert und unter Rühren auf -78°C gekühlt. Es werden 48 g Pentafluorethyliodid zugegeben, dann werden langsam 76 ml einer 1,5 m Lösung von Methyllithium-Lithiumbromidkomplex in Diethylether zugetropft. Es wird 2 Stunden bei -78°C gerührt und dann auf 21 gesättigte Natriumhydrogencarbonatlösung gegossen. Es wird mit Essigsäureethylester extrahiert, getrocknet und eingeengt.
Der Rückstand wird in 500 ml 95 %igem Methanol aufgenommen, mit 72 ml Methyliodid sowie 20 g Calciumcarbonat versetzt und 20 Stunden am Rückfluß erhitzt. Nach dem Abkühlen wird abgesaugt und das Filtrat wird zur Trockene eingeengt. Der Rückstand wird an Kieselgel chromatographiert und aus Essigsäureethylester umkristallisiert. Man erhält 17β-Hydroxy-17α-pentafluorethyl -androst-4-en-3-on.
¹H-NMR (CDCl₃): 0,99 (s,3H, H-18), 1,19 (s,3H, H-19), 5,74(s,1H, H-4)
¹⁹F-NMR : -77,3 (3F, CF₃), -119 (2F,CF₂)

### Beispiel 10

### 17β-Hydroxy-17α-pentafluorethyl-4-chlor-androst-4-en-3-on und 17β,4-Dihydroxy-17α-pentafluorethyl-androst-4-en-3-on

### Stufe 1

### 17β-Hydroxy-17α-pentafluorethyl-4ξ,5ξ-epoxy-androstan-3-on

Die Herstellung erfolgt analog 17β-Hydroxy-17α-trifluormethyl-4ξ,5ξ-epoxy-androstan-3-on. Der erhaltene Rückstand besteht aus einem Gemisch von 4α,5α- bzw. 4β,5β-Epoxiden und wird ohne weitere Reinigung in der Folgestufe eingesetzt.

### Stufe 2

### 17β-Hydroxy-17α-pentafluorethyl-4-chlor-androst-4-en-3-on

Die Herstellung erfolgt aus 17β-Hydroxy-17α-pentafluorethyl-4ξ,5ξ-epoxy-androstan-3-on analog zu 17β-Hydroxy-17α-trifluormethyl-4-chlor-androst-4-en-3-on.
¹H-NMR (CDCl₃): 0,99 (s, 3H, H-18), 1,23 (s, 3H, H-19)
¹⁹F-NMR: -77,4 (3F, CF₃), -119.2 (2F,CF₂)

### Stufe 3

### 17β,4-Dihydroxy-17α-pentafluorethyl-androst-4-en-3-on

Die Herstellung erfolgt aus 17β-Hydroxy-17α-pentafluorethyl-4ξ,5ξ-epoxy-androstan-3-on analog zu 17β,4-Dihydroxy-17α-trifluormethyl-androst-4-en-3-on.
¹H-NMR (CDCl₃): 0,98 (s, 3H, H-18), 1,18 (s, 3H, H-19), 6,09 (s, 1H, 4-OH)
¹⁹F-NMR : -77,4 (3F, CF₃), -119.5 (2F,CF₂)

### Beispiel 11

### 17β-Hydroxy-17α-pentafluorethyl-7α-methyl-androst-4-en-3-on

### Stufe 1

### 17β-Hydroxy-17α-pentafluorethyl-androst-4,6-dien-3-on

1 g 17β-Hydroxy-17α-pentafluorethyl-androst-4-en-3-on wird mit 1,2 g Chloranil in 50 ml tert.-Butanol 30 Minuten am Rückfluss gekocht. Man läßt abkühlen und engt zur Trockene ein. Der Rückstand wird an Kieselgel chromatographiert.
¹H-NMR (CDCl₃): 1,04 (s, 3H, H-18), 1,12 (s, 3H, H-19), 5,68 (s, 1H, H-4), 6,11 (m, 2H, H-6, H-7)
¹⁹F-NMR : -77,4 (3F, CF₃), -119,0 (2F, CF₂)
Zu einer Lösung von Methylmagesiumiodid (bereitet aus 5 g Magnesium und 13 ml Methyliodid in 150 ml Diethylether) werden 160 ml THF gegeben, man kühlt auf -5°C und gibt 2 g Kupferacetat-Monohydrat, gelöst in 100 ml THF, zu. Es wird auf -20°C gekühlt und dann wird eine Lösung von 10 g 17β-Hydroxy-17α-pentafluorethyl-androst-4,6-dien-3-on in 120 ml THF zugetropft. Nach 2 Stunden gießt man auf Eiswasser/2N Schwefelsäure und extrahiert mit 3 mal 80 ml Essigester. Der organische Extrakt wird getrocknet und eingeengt. Der Rückstand wird an Kieselgel chromatographiert. Zur weiteren Reinigung wird aus Essigester umkristallisiert.
¹H-NMR (CDCl₃): 0.78 (d J=8Hz,3H,H-7Me) 1.01 (s,3H, H-18), 1.21 (s,3H, H-19), 5.74 (s,1 H,H-4)
¹⁹F-NMR: -77.4 (3F,CF3), -119.3 (2F,CF2)

### Beispiel 12

### 17β-Hvdroxy-17α-Dentafluorethyl-estr-4-en-3-on

20 g 3,3-Dimethoxy-estr-5(10)-en-17-on werden in 600 ml Diethylether gelöst und unter Rühren auf -78°C gekühlt. Es werden 48 g Pentafluorethyliodid zugegeben, dann werden langsam 76 ml einer 1,5 m Lösung von Methyllithium-Lithiumbromidkomplex in Diethylether zugetropft. Es wird 2 Stunden bei -78°C gerührt und dann auf 21 gesättigte Natriumhydrogencarbonatlösung gegossen. Es wird mit Essigsäureethylester extrahiert, getrocknet und eingeengt.
Der Rückstand wird an Kieselgel chromatographiert und aus Essigsäureethylester umkristallisiert. Man erhält 17β-Hydroxy-17α-pentafluorethyl-estr-4-en-3-on.
¹H-NMR (CDCl₃): 1,02 (s, 3H, H-18), 5,83 (s, 1H, H-4)
¹⁹F-NMR : -77,3 (3F, CF₃), -119,2 (2F, CF₂)

### Beispiel 13

### 17β-Hydroxy-17α-pentafluorethyl-7α-methyl-estr-4-en-3-on

3,3-Ethylendithio-7α-methyl-estr-4-en-17-on (siehe Herstellung von 17β-Hydroxy-17α-trifluormethyl-7α-methyl-estr-4-en-3-on) wird analog wie voranstehend mit Pentafluorethyliodid/Methyllithium-Lithiumbromidkomplex umgesetzt. Das Rohprodukt wird in 500 ml 95 %-igem Methanol aufgenommen, mit 72 ml Methyliodid sowie 20 g Calciumcarbonat versetzt und 20 Stunden am Rückfluß erhitzt. Nach dem Abkühlen wird abgesaugt und das Filtrat wird zur Trockene eingeengt. Der Rückstand wird an Kieselgel chromatographiert und aus Essigsäureethylester umkristallisiert. Man erhält 17β-Hydroxy-17α-pentafluorethyl-7α-methyl-estr-4-en-3-on.
¹H-NMR (CDCl₃): 0,77 (d, J = 8 Hz, 3H, H-7Me), 1,02 (s, 3H, H-18), 5,84 (s, 1H, H-4)
¹⁹F-NMR : -77,3 (3F, CF₃), -119,2 (2F, CF₂)

### Beispiel 14

### 17β,4-Dihydroxy-17α-pentafluorethyl- estr -4-en-3-on

### Stufe 1

### 17β-Hyrdroxy-17α-pentafluorethyl-4ξ,5ξ-epoxy -estran-3-on

Die Herstellung erfolgt analog 17β-Hydroxy-17α-trifluormethyl-4ξ,5ξ-epoxy-androstan-3-on. Der erhaltene Rückstand besteht aus einem Gemisch von 4α,5α- bzw. 4β,5β-Epoxiden und wird ohne weitere Reinigung in der Folgestufe eingesetzt.

### Stufe 2

### 17β,4-Dihydroxy-17α-Pentafluorethyl-estr-4-en-3-on

Die Herstellung erfolgt aus 17β-Hydroxy-17α-pentafluorethyl-4ξ,5ξ-epoxy-estran-3-on analog zu 17β,4-Dihydroxy-17α-trifluormethyl-androst-4-en-3-on.
¹H-NMR (CDCl₃) : 1,00 (s, 3H, H-18), 6,10 (s, 1H, 4-OH)
¹⁹F-NMR : -77,3 (3F, CF₃), -119,2 (2F, CF₂)

### Beispiel 15

### 17β-Hydroxy-17α-pentafluorethyl-4-chlor- estr -4-en-3-on

Die Herstellung erfolgt aus 17β-Hydroxy-17α-pentafluorethyl-4ξ,5ξ-epoxy-estran-3-on analog zu 17β-Hydroxy-17α-trifluormethyl-4-chlor-androst-4-en-3-on.
¹H-NMR (CDCl₃) : 1,01 (s, 3H, H-18)
¹⁹F-NMR : -77,3 (3F, CF₃), -119,2 (2F, CF₂)

### Beispiel 16

### 17β-Hydroxy-17α- trifluormethyl-5α-androstan-3-on

### Stufe 1

### 3β-Acetoxy-17β-trimethylsilyloxy-17α-trifluormethyl-5α-androstan

10 g 3β-Acetoxy-epiandrosteron werden in 300 ml THF gelöst und mit 0,5 g Tetrabutylammoniumfluorid versetzt. Unter Rühren bei Raumtemperatur werden langsam 15 ml Trifluormethyltrimethylsilan zugetropft und es wird 3 Stunden gerührt. Dann gibt man 200 ml halbkonzentrierte Natriumhydrogencarbonatlösung zu und destilliert das THF im Vakuum ab. Der Rückstand wird dreimal mit 100 ml Essigsäureethylester extrahiert. Die vereinigten organischen Extrakte werden getrocknet, eingeengt und an Kieselgel chromatographiert. Man erhält 9 g 3β-Acetoxy-17β-trimethylsilyloxy-17α-trifluormethyl-5α-androstan.

### Stufe 2

### 3β-Acetoxy-17β-hydroxy-17α-trifluormethyl-5α-androstan

9 g 3β-Acetoxy-17β-trimethylsilyloxy-17α-trifluormethyl-5α-androstan werden in 100 ml THF gelöst und bei Raumtemperatur mit 20 ml 30 %-iger Fluorwasserstoffsäure versetzt. Nach 3 Stunden gießt man auf 200 ml 12 %-ige Ammoniaklösung, extrahiert mit 3 mal 100 ml Essigester, trocknet die organischen Extrakte und engt ein. Man erhält 7 g 3β-Acetoxy-17β-hydroxy-17α-trifluormethyl-5α-androstan.

### Stufe 3

### 3β,17β-Dihydroxy-17α-trifluormethyl-5α-androstan

7 g 3β-Acetoxy-17β-hydroxy-17α-trifluormethyl-5α-androstan wird in 300 ml Methanol gelöst und mit 6 g Kaliumhydroxid versetzt. Nach 30 Minuten Rühren bei Raumtemperatur wird mit 2n Salzsäure neutralisiert und das Methanol wird im Vakuum abgezogen. Der Rückstand wird mit 4 mal 100 ml Essigsäureethylester ausextrahiert und die vereinigten organischen Extrakte werden getrocknet und eingeengt. Man erhält 4,5 g 3β, 17β-Dihydroxy-17α-trifluormethyl-5α-androstan.

### Stufe 4

### 17β-Hydroxy-17α-trifluormethyl-5α-androstan -3-on

Das in Stufe 3 erhaltene Produkt wird in 50 ml Aceton mit 9 ml 8 n Chromschwefelsäure bei 0 °C oxidiert. Nach beendeter Reaktion gibt man 2 ml Methanol sowie 50 ml Wasser zu und zieht das Aceton im Vakuum ab, wobei das Produkt ausfällt. Es wird abgesaugt und mit Wasser gewaschen. Zur Reinigung wird chromatographiert und aus Essigsäureethylester kristallisiert. Man erhält 17β-Hydroxy-17α-trifluormethyl-5α-androstan-3-on.
¹H-NMR (CDCl₃): 0,96 (s, 3H, H-18), 1,02 (s, 3H, H-19)
¹⁹F-NMR : -75,4

### Beispiel 17

### 17β-Hydroxy-17α-pentafluorethyl-5α-androstan-3-on

### Stufe 1

### 3β-Trimethylsilyloxy-5α-androstan-17-on

10 g Epiandrosteron werden in 75 ml DMF und 20 ml Pyridin gelöst und mit 10 ml Trimethylchlorsilan versetzt. Nach 3 Stunden wird in 300 ml gesättigte Natriumhydrogencarbonatlösung gegossen. Man saugt ab, wäscht mit Wasser und erhält 15 g 3β-Trimethylsilyloxy-5α-androstan-17-on.

### Stufe 2

### 3β, 17β-Dihydroxy-17α-pentafluorethyl-5α-androstan

15 g 3β-Trimethylsilyloxy-5α-androstan-17-on werden in 300 ml Diethylether gelöst und auf - 78°C gekühlt. Es werden 14 g Pentafluorethyliodid zugegeben und dann 38 ml einer 1,5 m Lösung von Methyllithium-Lithiumbromidkomplex in Diethylether zugetropft. Man läßt 1 Stunde Rühren und gießt auf 1 I gesättigte Natriumhydrogencarbonatlösung, extrahiert mit Essigsäureethylester und engt ein. Der Rückstand wird in 100 ml THF aufgenommen und mit 8 g Tetrabutylammoniumfluorid versetzt. Nach 30 Minuten werden 200 ml gesättigte Natriumhydrogencarbonatlösung zugegeben und das THF wird im Vakuum abgezogen, wobei die Substanz ausfällt. Es wird abgesaugt und mit Wasser gewaschen. Man erhält 13 g 3β,17β-Dihydroxy-17α-pentafluorethyl-5α-androstan.

### Stufe 3

### 17β-Hydroxy-17α-pentafluorethyl-5α-androstan-3-on

10 g 3β,17β-Dihydroxy-17α-pentafluorethyl-5α-androstan werden in 100 ml Aceton mit 16 ml 8n Chromschwefelsäure bei 0 °C oxidiert. Man gibt 3 ml Methanol,100 ml Wasser zu und zieht das Aceton im Vakuum ab, wobei das Produkt auskristallisiert. Es wird abgesaugt und mit Wasser gewaschen. Man erhält 17β-Hydroxy-17α-pentafluorethyl-5α-androstan-3-on.
¹H-NMR (CDCl₃) : 0,96 (s, 3H, H-18), 1,02 (s, 3H, H-19)
¹⁹F-NMR : -77,3 (3F, CF₃), -119,3 (2F,CF₂)

### Beispiel 18

### 17β-Hydroxy-17α- trifluormethyl-2-hydroxymethylen-5α-androstan-3-on

4 g 17β-Hydroxy-17α-trifluormethyl-5α-androstan-3-on werden in 150 ml Toluol mit 3,2 g Natriumhydrid und 8 ml Ameisensäurethylester versetzt. Nach 24 Stunden wird vorsichtig mit Wasser hydrolysiert. Man säuert mit 5 n Salzsäure an, trennt die organische Phase ab, trocknet und engt ein. Zur Reinigung wird an Kieselgel - chromatographiert und aus Aceton/Hexan kristallisiert. Man erhält 17β-Hydroxy-17α-trifluormethyl-2-hydroxymethylen-5α-androstan-3-on.
¹H-NMR (CDCl₃): 0,76 (s, 3H, H-18), 0,97 (s, 3H, H-19), 8,62 (m, 1H, H-2-CHO)
¹⁹F-NMR : -75,2

### Beispiel 19

### 17β-Hydroxy-17α-pentafluorethyl-2-hydroxymethylen -5α-androstan-3-on

Die Verbindung wird analog Beispiel 17 [?] aus 17β-Hydroxy-17α-pentafluorethyl-5α-androstan-3-one erhalten.
¹H-NMR (CDCl₃) : 0,77 (s, 3H, H-18), 0.96 (s, 3H, H-19)), 8,62 (m, 1H, H-2-CHO)
¹⁹F-NMR: -77,3 (3F, CF₃), -119,2 (2F, CF₂)

### Beispiel 20

### 17β-Hydroxy-17α- trifluormethyl-[3,2c] pyrazol -5α-androstan

1 g 17β-Hydroxy-17α-trifluormethyl-2-hydroxymethylen-5α-androstan-3-on wird in 50 ml Ethanol unter Zusatz von 0,3 ml Hydrazinhydrat 30 Minuten am Rückfluss erhitzt. Dann wird eigeengt, mit Wasser gefällt und abgesaugt. Das Produkt wird durch Kristallisation aus tert.-Butyl-methylether/Hexan gereinigt. Man erhält 17β-Hydroxy-17α-trifluormethyl-[3,2c]pyrazol-5α-androstan
¹H-NMR (CDCl₃) : 0,74 (s, 3H, H-18), 0,96 (s, 3H, H-19)
¹⁹F-NMR : -75,3

### Beispiel 21

### 17β-Hydroxy-17α-pentafluorethyl-[3,2c]pyrazol-5α-androstan

Wird analog Beispiel 19 Verbindung aus 17β-Hydroxy-17α-pentafluorethyl-2-hydroxymethylen-5α-androstan-3-on erhalten.
¹H-NMR (CDCl₃): 0,74 (s, 3H, H-18), 0,96 (s, 3H, H-19)
¹⁹F-NMR : -77,3 (3F, CF₃), -119.3 (2F, CF₂)

### Beispiel 22

### 17β-Hydroxy-17α-trifluormethyl-5α-androst-1-en-3-on

10 g 17β-Hydroxy-17α-trifluormethyl-5α-androstan-3-on werden unter Rühren in 200 ml THF mit 9 g Pyridinium-Hydrobromid-Perbromid versetzt. Nach 15 Minuten gibt man 250 ml gesättigte Natriumhydrogencarbonatlösung zu, extrahiert mit Chloroform, trocknet und engt ein. Der Rückstand wird mit 10 g Lithiumcarbonat und 20 g Lithiumbromid in 100 ml DMF 6 Stunden am Rückfluß erhitzt. Man läßt abkühlen, verdünnt mit 500 ml Toluol, wäscht mit Wasser, trocknet und engt ein. Zur Reinigung wird an Kieselgel chromatographiert und aus Essigsäureethylester umkristallisiert. Man erhält 17β-Hydroxy-17α-trifluormethyl-5α-androst-1-en-3-on.
¹H-NMR (CDCl₃) : 0,98 (s, 3H, H-18), 1,02 (s, 3H, H-19), 5,85 (d, J = 10 Hz, 1H, H-2), 7,12 (d, J = 10 Hz, 1H, H-1)
¹⁹F-NMR : -75,3

### Beispiel 23

### 17β-Hydroxy-17α-pentafluorethyl-5α-androst-1-en-3-on

Wird analog Beispiel 21 aus 17β-Hydroxy-17α-pentafluorethyl-5α-androstan-3-on erhalten.
¹H-NMR (CDCl₃) : 0,98 (s, 3H, H-18), 1,02 (s, 3H, H-19), 5.85 (d, J = 10 Hz, 1H, H-2), 7,12 (d, J = 10 Hz, 1H, H-1)
¹⁹F-NMR : -77,3 (3F,CF₃), -119,2 (2F,CF₂)

### Beispiel 24

### 17β-Hydroxy-17α- trifluormethyl-2-oxa-5α-androstan-3-on

4 g 17β-Hydroxy-17α-trifluormethyl-5α-androst-1-en-3-on werden in 200 ml 90 %-iger Essigsäure mit 30 g Bleitetraacetat und 280 mg Osmiumtetroxid umgesetzt. Nach 24 Stunden bei Raumtemperatur wird mit 500 ml Wasser verdünnt und dreimal mit Chloroform extrahiert. Die vereinigten organischen Phasen werden mit 2n Natronlauge alkalisiert und dreimal mit 200 ml 2n Natronlauge extrahiert. Die vereinigten wäßrigen Phasen werden mit 5n Salzsäure angesäuert und dreimal mit Chloroform extrahiert. Die vereinigten organischen Phasen werden getrocknet und eingeengt.
Der Rückstand wird in 80 ml THF und 80 ml Methanol gelöst. Unter Rühren werden nacheinander eine Lösung von 1 g Natriumhydrogencarbonat in 75 ml Wasser und 4,2 g Natriumborhydrid zugegeben. Nach 2 Stunden wird mit konz. Salzsäure angesäuert, mit Essigsäureethylester extrahiert, getrocknet und eingeengt. Zur Reinigung wird an Kieselgel chromatographiert und aus Essigsäureethylester umkristallisiert. Man erhält 17β-Hydroxy-17α-trifluormethyl-2-oxa-5α-androstan-3-on.
¹H-NMR (CDCl₃) : 0,94 (s, 3H, H-18), 1,00 (s, 3H, H-19), 2,22 (dd, J = 19,1, 13,1 Hz, 1H, H-4), 2,53 (dd, J = 18,7, 5,8 Hz, 1 H, H-4), 3,92 (d, J = 10 Hz, 1 H, H-1), 4,21 (d, J = 10 Hz, 1H, H-1)
¹⁹F-NMR : -75,4

### Beispiel 25

### 17β-Hydroxy-17α-pentafluorethyl-2-oxa-5α-androstan-3-on

Wird analog Beispiel 23 aus 17β-Hydroxy-17α-pentafluorethyl-5α-androst-1-en-3-on erhalten.
¹H-NMR (CDCl₃) : 0,94 (s, 3H, H-18), 1,00 (s, 3H, H-19), 2,22 (dd, J = 19,1, 13,0 Hz, 1 H, H-4), 2,53 (dd, J = 19,1, 6,2 Hz, 1 H, H-4), 3,93 (d, J = 10 Hz, 1 H, H-1), 4,22 (d, J = 10 Hz, 1H, H-1)
¹⁹F-NMR : -77,3 (3F,CF₃), -119,2 (2F,CF₂)

### Beispiel 26

### 17β-Hydroxy-17α- trifluormethyl-5α-androst-2-en

### Stufe 1

### 17β-Trimethylsilyloxy-17α-trifluormethyl-5α-androst-2-en

10 g 5α-Androst-2-en-17-on (Herstellung nach US-A-3,098,851) werden in 300 ml THF gelöst und mit 0,5 g Tetrabutylammoniumfluorid versetzt. Unter Rühren bei Raumtemperatur werden langsam 15 ml Trifluormethyltrimethylsilan zugetropft und es wird 3 Stunden gerührt. Dann gibt man 200 ml halbkonzentrierte Natriumhydrogencarbonatlösung zu und destilliert das THF im Vakuum ab. Der Rückstand wird dreimal mit 100 ml Essigsäureethylester extrahiert. Die vereinigten organischen Extrakte werden getrocknet, eingeengt und an Kieselgel chromatographiert. Man erhält 10 g 17β-Trimethylsilyloxy-17α-trifluormethyl-5α-androst-2-en.

### Stufe 2

### 17β-Hydroxy-17α-trifluormethyl-5α-androst-2-en

10 g 17β-Trimethylsilyloxy-17α-trifluormethyl-5α-androst-2-en werden in 100 ml THF gelöst und bei Raumtemperatur mit 20 ml 30 %-iger Fluorwasserstoffsäure versetzt. Nach 3 Stunden gießt man auf 200 ml 12 %-ige Ammoniaklösung, extrahiert mit 3 mal 100 ml Essigester, trocknet die organischen Extrakte und engt ein. Nach Kristallisation aus Methanol erhält man 17β-Hydroxy-17α-trifluormethyl-5α-androst-2-en.
¹H-NMR (CDCl₃): 0,76 (s, 3H, H-18), 0,93 (s, 3H, H-19), 5,60 (m, 2H, H-2, H-3) ¹⁹F-NMR : -75,1

### Beispiel 27

### 17β-Hydroxy-17α-pentafluorethyl-5α-androst-2-en

15 g 5α-Androst-2-en-17-on werden in 300 ml Diethylether gelöst und auf - 78°C gekühlt. Es werden 14 g Pentafluorethyliodid zugegeben und dann 38 ml einer 1,5 m Lösung von Methyllithium-Lithiumbromidkomplex in Diethylether zugetropft. Man läßt 1 Stunde Rühren und gießt auf 1l gesättigte Natriumhydrogencarbonatlösung, extrahiert mit Essigsäureethylester und engt ein. Der Rückstand wird an Kieselgel chromatographiert und man erhält 17β-Hydroxy-17α-pentafluorethyl-5α-androst-2-en. ¹H-NMR (CDCl₃) : 0,76 (s, 3H, H-18), 0,95 (s, 3H, H-19), 5,62 (m, 2H, H-2, H-3)
¹⁹F-NMR : -77,5 (3F, CF₃), -119,3 (2F, CF₂)

## Patentansprüche

1. 17α-Fluoralkylsteroide der allgemeinen Formel (I) 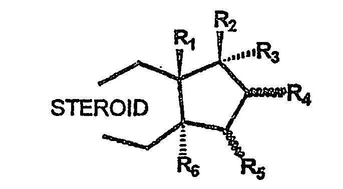
worin R¹ für eine C₁₋₄-Alkylgruppe,
R² für eine Hydroxygruppe, eine Gruppe OC(O)-R²⁰ oder OR²¹ steht, wobei R²⁰ und R²¹ eine C₁₋₁₂-Alkylgruppe, eine C₃₋₈-Cycloalkylgruppe, eine Arylgruppe mit 6 bis 15 C-Atomen die substituiert sein kann oder eine Aryl-C₁₋₄-Alkylgruppe , die zusammen 7 bis 15 C-Atome aufweist und am Arylrest substituiert sein kann bedeuten,
R³ für einen Rest der Formel CnF2n+1, wobei n=1, 2, 3, 4, 5 oder 6,
R⁴ und R⁵ jeweils für ein Wasserstoffatom, gemeinsam für eine Doppelbindung oder eine Methylenbrücke,
R⁵ und R⁶ je für ein Wasserstoffatom, gemeinsam für eine Doppelbindung oder eine Methylenbrücke,
STEROID für ein steroidales ABC-Ringsystem der Teilformeln A, B, C, D, E und F stehen: 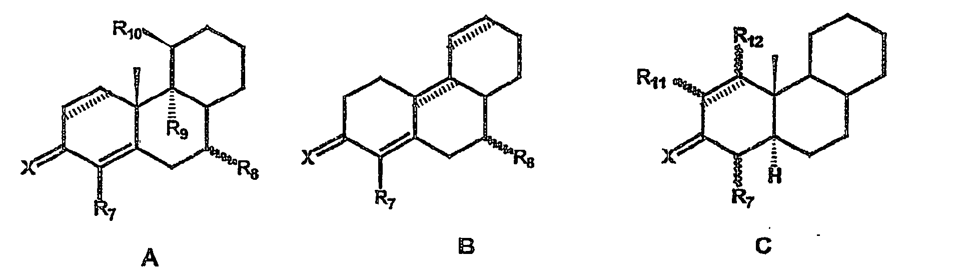 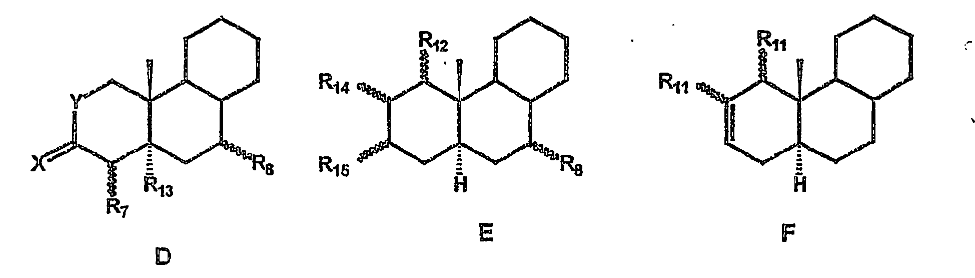 wobei sich in A und C in 1,2-Stellung eine zusätzliche Doppelbindung und sich in B in 8,9 Stellung und 11,12 Stellung eine oder zwei zusätzliche Doppelbindungen befinden können,
R⁷ ein Wasserstoffatom, ein Halogenatom, eine Hydroxylgruppe oder eine Trifluormethylgruppe bedeutet,
X ein Sauerstoffatom, oder zwei Wasserstoffatome bedeutet,
R⁸ ein Wasserstoffatom , eine Methyl- oder Ethylgruppe bedeutet,
R⁹ ein Wasserstoffatom oder ein Halogenatom bedeutet oder gemeinsam mit R¹⁰ für eine Doppelbindung steht,
R¹⁰ ein Wasserstoffatom, eine Hydroxylgruppe, eine Methyl- oder Ethylgruppe bedeutet oder gemeinsam mit R⁹ für eine Doppelbindung steht,
R¹¹ ein Wasserstoffatom, eine C₁₋₄-Alkylgruppe, eine Nitrilgruppe, eine Hydroxymethylen- oder Formylgruppe bedeutet,
R¹² ein Wasserstoffatom, eine C₁₋₄-Alkylgruppe oder eine Nitrilgruppe bedeutet,
R¹¹ und R¹² neben den vorstehend genannten Bedeutungen zusammen eine Methylenbrücke bedeuten,
R¹³ ein Wasserstoffatom oder zusammen mit R⁷ eine Doppelbindung bedeutet,
R¹⁴ und R¹⁵ zusammen für eine Doppelbindung, einen Oxiranring, einen Thiiranring, einen [2,3c]Oxadiazolring, einen [3,2c]Isoxazolring oder einen [3,2c]Pyrazolring stehen,
Y für ein Sauerstoff- oder Stickstoffatom steht,
wobei die geschlängelten Linien an R⁷, R⁸, R¹¹, R¹², R¹³, R¹⁴ und R¹⁵ bedeuten, daß diese Substituenten α- oder β-ständig sein können und die folgenden Verbindungen ausgenommen sind:
17β-Hydroxy-17α-trifluormethyl-androst-4-en-3-on
17β-Hydroxy-17α-trifluormethyl-estr-4-en-3-on,
17β-Hydroxy-17α-trifluormethyl-estr-4,9-dien-3-on,
17β-Hydroxy-17α-trifluormethyl-estr-4, 9 ,11-trien-3-on,
13-Ethyl 17β-hydroxy-17α-triftuormethyl-gon-4-en-3-on.
13-Ethyl-17β-hydroxy-17α-trifluormethyl-gon-4,9-dien-3-on und
13-Ethyl-17β-hydroxy-17α-trifluormethyl-gon-4,9,11-trien-3-on.

2. 17α-Fluoralkylsteroide nach Anspruch 1, wobei STEROID für ein steroidales Ringsystem der Teilformel A steht.

3. 17α-Fluoralkylsteroide nach Anspruch 1, wobei STEROID für ein steroidales Ringsystem der Teilformel B steht.

4. 17α-Fluoralkylsteroide nach Anspruch 1, wobei STEROID für ein steroidales Ringsystem der Teilformel C steht.

5. 17α-Fluoralkylsteroide nach Anspruch 1, wobei STEROID für ein steroidales Ringsystem der Teilformel D steht.

6. 17α-Fluoralkylsteroide nach Anspruch 1, wobei STEROID für ein steroidales Ringsystem der Teilformel E steht.

7. 17α-Fluoralkylsteroide nach Anspruch 1, wobei STEROID für ein steroidales Ringsystem der Teilformel F steht.

8. 17α-Fluoralkylsteroide nach einem der Ansprüche 1 bis 7, wobei R¹ eine Methylgruppe darstellt.

9. 17α-Fluoralkylsteroide nach einem der Ansprüche 1 bis 8, wobei R² eine Hydroxygruppe, eine Formyloxygruppe, Acetyloxygruppe, Propionyloxygruppe, Butyryloxygruppe, [(trans-4-Butylcyclohexyl)carbonyl]oxygruppe, Phenylpropionyloxygruppe, iso-Butyryloxygruppe, Heptanyloxygruppe oder Undecanyloxygruppe bedeutet.

10. 17α-Fluoralkylsteroide nach einem der Ansprüche 1 bis 9, wobei R³ eine Trifluormethylgruppe oder eine Pentafluorethylgruppe darstellt.

11. 17α-Fluoralkylsteroide nach einem der Ansprüche 1 bis 3 sowie 8 bis 10, wobei R₈ eine Methylgruppe ist.

12. 17α-Fluoralkylsteroide nach einem der Ansprüche 1 bis 5 sowie 8 bis 11, wobei R₇ ein Fluor-, Chlor oder Bromatom oder eine Trifluormethyl- oder Hydroxygruppe darstellt.

13. 17α-Fluoralkylsteroide nach einem der Ansprüche 1, 2 sowie 8 bis 11, wobei R¹⁰ eine Hydroxygruppe darstellt.

14. 17α-Fluoralkylsteroide nach einem der Ansprüche 1, 2 sowie 8 bis 12, wobei R⁹ ein Fluoratom ist.

15. 17α-Fluoralkylsteroide nach einem der Ansprüche 1, 4, 7 und 12, wobei R¹¹ eine Hydroxymethylengruppe oder Formylgruppe darstellt.

16. 17α-Fluoralkylsteroide nach einem der Ansprüche 1, 5, 11 und 12, wobei Y ein Sauerstoffatom ist.

17. 17α-Fluoralkylsteroide nach Anspruch 1, nämlich
17β-Hydroxy-17α-trifluormethyl-7α-methyl-androst-4-en-3-on,
17β,4-Dihydroxy-17α-trifluormethyl-androst-4-en-3-on,
17β-Hydroxy-17α-trifluormethyl-4-chlor-androst-4-en-3-on,
17β-Hydroxy-17α-trifluormethyl-4-brom-androst-4-en-3-on,
17β-Hydroxy-17α,4-bis(trifluormethyl)-androst-4-en-3-on,
17β,11β-Dihydroxy-17α-trifluormethyl-androst-4-en-3-on,
17β,11β-Dihydroxy-17α-trifluormethyl-9α-fluor-androst-4-en-3-on,
17β-Hydroxy-17α-trifluormethyl-androst-1,4-dien-3-on,
17β-Hydroxy-17α-trifluormethyl-4-chlor-androst-1,4-dien-3-on,
17β,4-Dihydroxy-17α-trifluormethyl-androst-1,4-dien-3-on,
17β-Hydroxy-17α-trifluormethyl-7α-methyl-androst-1,4-dien-3-on,
17β-Hydroxy-17α-trifluormethyl-7α-methyl-4-chlor-androst-1,4-dien-3-on,
17β-Hydroxy-17α-pentafluorethyl-androst-4-en-3-on,
17β-Hydroxy-17α-pentafluorethyl-7α-methyl-androst-4-en-3-on,
17β,4-Dihydroxy-17α-pentafluorethyl-androst-4-en-3-on,
17β-Hydroxy-17α-pentafluorethyl-4-chlor-androst-4-en-3-on,
17β-Hydroxy-17α-pentafluorethyl-4-brom-androst-4-en-3-on,
17β-Hydroxy-17α-pentafluorethyl-4-trifluormethyl-androst-4-en-3-on,
17β,11β-Dihydroxy-17α-pentafluorethyl-androst-4-en-3-on,
17β,11β-Dihydroxy-17α-pentafluorethyl-9α-fluor-androst-4-ein-3-on,
17β-Hydroxy-17α-pentafluorethyl-androst-1,4-dien-3-on,
17β-Hydroxy-17α-pentafluorethyl-4-chlor-androst-1,4-dien-3-on,
17β,4-Dihydroxy-17α-pentafluorethyl-androst-1,4-dien-3-on,
17β-Hydroxy-17α-pentafluorethyl-4-trifluormethyl-androst-1,4-dien-3-on,
17β-Hydroxy-17α-pentafluorethyl-7α-methyl-androst-1 ,4-dien-3-on,
17β-Hydroxy-17α-pentafluorethyl-7α-methyl-4-chlor-androst-1,4-dien-3-on,
17β-Hydroxy-17α-trifluormethyl-7α-methyl-estr-4-en-3-on,
17β,4-Dihydroxy-17α-trifluormethyl-estr-4-en-3-on,
17β-Hydroxy-17α-trifluormethyl-4-chlor-estr-4-en-3-on,
17β-Hydroxy-17α-trifluormethyl-4-brom-estr-4-en-3-on,
17β-Hydroxy-17α,4-bis(trifluormethyl)-estr-4-en-3-on,
17β-Hydroxy-17α-trifluormethyl-7α-methyl-estr-4,9-dien-3-on,
17β-Hydroxy-17α-trifluormethyl-7α-methyl-estr-4,9,11-trien-3-on,
17β-Hydroxy-17α-pentaftuorethyl-estr-4-en-3-on,
17β-Hydroxy-17α-pentafluorethyl-7α-methyl-estr-4-en-3-on,
17β,4-Dihydroxy-17α-pentafluorethyl-estr-4-en-3-on,
17β-Hydroxy-17α-pentafluorethyl-4-chlor-estr-4-en-3-on,
17β-Hydroxy-17α-pentafluorethyl-4-brom-estr-4-en-3-on,
17β-Hydroxy-17α-pentafluorethyl-4-trifluormethyl-estr-4-en-3-on,
17β-Hydroxy-17α-pentafluorethyl-estr-4,9-dien-3-on,
17β-Hydroxy-17α-pentafluorethyl-estr-4,9,11-trien-3-on,
17β-Hydroxy-17α-pentafluorethyl-7α-methyl-estr-4,9-dien-3-on,
17β-Hydroxy-17α-pentafluorethyl-7α-methyl-estr-4,9,11-trien-3-on,
13-Ethyl-17β-hydroxy- 17α-trifluormethyl-4-chlor-gon-4-en-3-on,
13-Ethyl-17β,4-dihydroxy-17α-trifluormethyl-gon-4-en-3-on,
13-Ethyl-17β-hydroxy-17α-trifluormethyl-7α-methyl-gon-4-en-3-on,
13-Ethyl-17β-hydroxy-17α-trifluormethyl-7α-methyl-gon-4,9-dien-3-on,
13-Ethyl-17β-hydroxy-17α-trifluormethyl-7α-methyl-gon-4,9,11-trien-3-on,
13-Ethyl-17β-hydroxy-17α-pentafluorethyl-gon-4-en-3-on,
13-Ethyl-17β-hydroxy-17α-pentafluorethyl-7α-methyl-gon-4-en-3-on,
13-Ethyl17β,4-Dihydroxy-17α-pentafluorethyl-gon-4-en-3-on,
13-Ethyl-17β-hydroxy-17α-pentafluorethyl-4-chlor-gon-4-en-3-on,
13-Ethyl-17β-hydroxy-17α-pentafluorethyl-4-brom-gon-4-en-3-on,
13-Ethyl-17β-hydroxy-17α-pentafluorethyl-4-triftuormethyl-gon-4-en-3-on,
13-Ethyl-17β-hydroxy-17α-pentafluorethyl-gon-4,9-dien-3-on,
13-Ethyl-17β-hydroxy-17α-pentafluorethyl-gon-4,9,11-trien-3-on,
13-Ethyl-17β-hydroxy-17α-pentafluorethyl-7α-methyl-gon-4,9-dien-3-on,
13-Ethyl-17β-hydroxy-17α-pentafluorethyl-7α-methyl-gon-4, 9, 11-trien-3-on,
17β-Hydroxy-17α-trifluormethyl-5α-androstan-3-on,
17β-Hydroxy-17α-pentafluorethyl-5α-androstan-3-on,
17β-Hydroxy-17α-trifluormethyl-7α-methyl-5α-androstan-3-on,
17β-Hydroxy-17α-pentafluorethyl-7α-methyl-5α-androstan-3-on,
17β-Hydroxy-17α-trifluormethyl-2-hydroxymethylen-5α-androstan-3-on,
17β-Hydroxy-17α-pentafluorethyl-2-hydroxymethylen-5α-androstan-3-on,
17β-Hydroxy-17α-trifluormethyl-2α-methyl-5α-androstan-3-on,
17β-Hydroxy-17α-pentafluorethyl-2α-methyl-5α-androstan-3-on,
17β-Hydroxy-17α-trifluormethyl-1α-methyl-5α-androstan-3-on,
17β-Hydroxy-17α-pentafluorethyl-1α-methyl-5α-androstan-3-on,
17β-Hydroxy-17α-trifluormethyl-5α-androst-2-en,
17β-Hydrouy-17α-pentafluorethyl-5α-androst-2-en,
17β-Hydroxy-17α-trifluormethyl-2-methyl-5α-androst-2-en,
17β-Hydroxy-17α-pentafluorethyl-2-methyl-5α-androst-2-en,
17β-Hydroxy-17α-trifluormethyl-2-cyano-5α-androst-2-en,
17β-Hydroxy-17α-pentafluorethyl-2-cyano-5α-androst-2-en,
17β-Hydroxy-17α-trifluormethyl-2-formyl-5α-androst-2-en,
17β-Hydroxy-17α-pentafluorethyl-2-formyl-5α-androst-2-en,
17β-Hydroxy-17α-trifluormethyl-[2,3c]oxadiazol-5α-androstan,
17β-Hydroxy-17α-pentafluorethyl-[2,3c]oxadiazol-5α-androstan,
17β-Hydroxy-17α-trifluormethyl-[3,2c]isoxazol-5α-androstan,
17β-Hydroxy-17α-pentafluorethyl-[3,2c]isoxazol-5α-androstan,
17β-Hydroxy-17α-trifluormethyl-[3,2c]pyrazol-5α-androstan,
17β-Hydroxy-17α-pentafluorethyl-[3,2c]pyrazol-5α-androstan,
17β-Hydroxy-17α-trifluormethyl-2β,3β-epithio-5α-androstan,
17β-Hydroxy-17α-pentafluorethyl-2β,3β-epithio-5α-androstan,
17β-Hydroxy-17α-trifluormethyl-2α,3α-epithio-5α-androstan,
17β-Hydroxy-17α-pentafluorethyl-2α,3α-epithio-5α-androstan,
17β-Hydroxy-17α-trifluormethyl-2-oxa-5α-androstan-3-on,
17β-Hydroxy-17α-pentafluorethyl-2-oxa-5α-androstan-3-on,
17β-Hydroxy-17α-trifluormethyl-5α-androst-1-en-3-on,
17β-Hydroxy-17α-pentafluorethyl-5α-androst-1-en-3-on,
17β-Hydroxy-17α-trifluormethyl-1-methyl-5α-androst-1-en-3-on,
17β-Hydroxy-17α-pentafluorethyl-1-methyl-5α-androst-1-en-3-on,
17β-Hydroxy-17α-trifluormethyl-2-methyl-5α-androst-1-en-3-on und
17β-Hydroxy-17α-pentafluorethyl-2-methyl-5α-androst-1-en-3-on.

18. Verfahren zur Herstellung von 17α-Fluoralkylsteroiden der allgemeinen Formel (I) nach Anspruch 1, **dadurch gekennzeichnet, daß** Verbindungen der allgemeinen Formel (II) 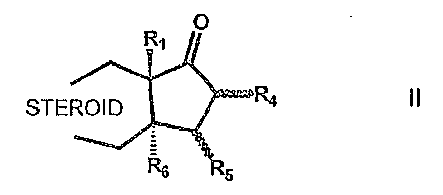 in der R¹, R⁴, R⁵, R⁶ und STEROID, die in Anspruch 1 gegebene Bedeutung haben, in Gegenwart von Fluorid mit Perfluoralkyltrialkylsilanen, (Alk)₃SiCnF2n+1 oder mit Fluoralkyllithium, LiCn2n+1, bzw. Fluoralkylgrignardreagenzien, ZMgCnF2n+1 umgesetzt werden, wobei n = 1, 2, 3, 4, 5 oder 6, ist, Z ein Chlor-, Brom- oder Iodatom ist und Alk ein C₁₋₄-Alkylrest ist.

19. Pharmazeutische Zusammensetzungen, die mindestens ein 17α-Fluoralkylsteroid der allgemeinen Formel (I) nach einem der Ansprüche 1 bis 17, gegebenenfalls zusammen mit pharmazeutisch verträglichen Hilfs- und Trägerstoffen enthalten.

20. 17α-Fluorallrylsteroide der Formel (I) nach einem der Ansprüche 1 bis 17 zur Anwendung als therapeutische Wirkstoffe.

21. Verwendung der 17α-Fluoralkylsteroide der allgemeinen Formel (I) 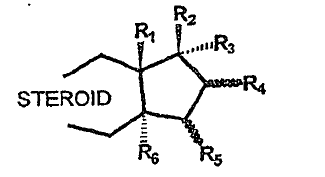
worin R¹ für eine C₁₋₄-Alkylgruppe,
R² für eine Hydroxygruppe, ein Gruppe OC(O)-R²⁰ oder OR²¹ steht, wobei R²⁰ und R²¹ eine C₁₋₁₂-Alkylgruppe, eine C₃₋₈-Cycloalkylgruppe, eine Arylgruppe mit 6 bis 15 C-Atome, die substituiert sein kann oder eine Aryl-C₁₋₄-Alkylgruppe die zusammen 7 bis 15 C-Atome aufweist und am Arylrest substituiert sein kann bedeuten,
R³ für einen Rest der Formel CnF2n+1, wobei n=1, 2, 3, 4, 5 oder 6,
R⁴ und R⁵ jeweils für ein Wasserstoffatom, gemeinsam für eine Doppelbindung oder eine Methylenbrücke,
R⁵ und R⁶ je für ein Wasserstoffatom, gemeinsam für eine Doppelbindung oder eine Methylenbrücke,
STEROID für ein steroidales ABC-Ringsystem der Teilformeln A, B, C, D, E und F stehen: 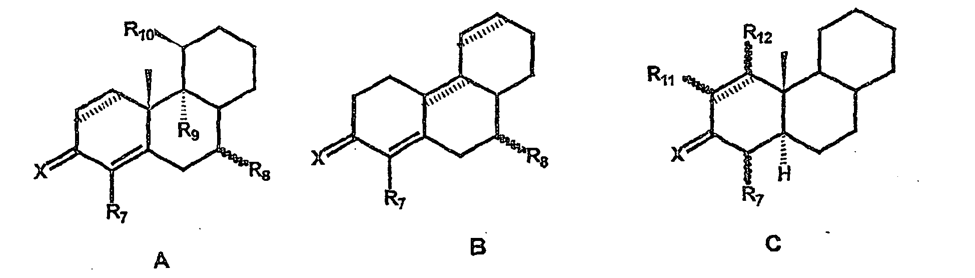 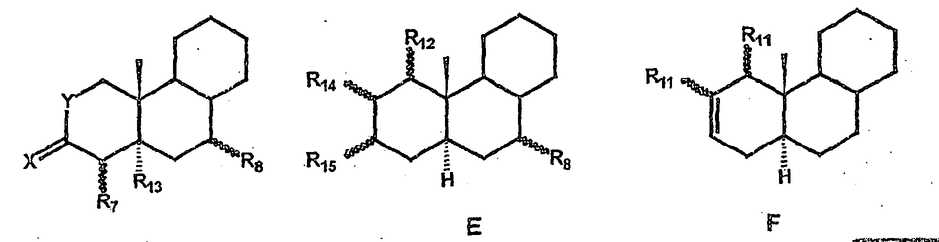 wobei sich in A und C in 1,2-Stellung eine zusätzliche Doppelbindung und sich in B in 8,9 Stellung und 11,12 Stellung eine oder zwei zusätzliche Doppelbindungen befinden können,
R⁷ ein Wasserstoffatom, ein Halogenatom, eine Hydroxylgruppe oder eine Trifluormethylgruppe bedeutet,
X ein Sauerstoffatom oder, zwei Wasserstoffatome bedeutet,
R⁸ ein Wasserstoffatom , eine Methyl- oder Ethylgruppe bedeutet,
R⁹ ein Wasserstoffatom oder ein Halogenatom bedeutet oder gemeinsam mit R¹⁰ für eine Doppelbindung steht,
R¹⁰ ein Wasserstoffatom, eine Hydroxylgruppe, eine Methyl- oder Ethylgruppe bedeutet oder gemeinsam mit R⁹ für eine Doppelbindung steht,
R¹¹ ein Wasserstoffatom, eine C₁₋₄-Alkylgruppe, eine Nitrilgruppe, eine Hydroxymethylen- oder Formylgruppe bedeutet,
R¹² ein Wasserstoffatom, eine C₁₋₄-Alkylgruppe oder eine Nitrilgruppe bedeutet,
R¹¹ und R¹² neben den vorstehend genannten Bedeutungen zusammen eine Methylenbrücke bedeuten,
R¹³ ein Wasserstoffatom oder zusammen mit R⁷eine Doppelbindung bedeutet,
R¹⁴ und R¹⁵ zusammen für eine Doppelbindung, einen Oxiranring, einen Thiiranring, einen [2,3c]Oxadiazolring, einen [3,2c]lsoxazolring oder einen [3,2c]Pyrazolring stehen,
Y für ein Sauerstoff- oder Stickstoffatom steht,
wobei die geschlängelten Linien an R⁷, R⁸, R¹¹, R¹² , R¹³, R¹⁴ und R¹⁵ bedeuten, daß diese Substituenten α- oder β-ständig sein können und die folgenden Verbindungen ausgenommen sind:
17β-Hydroxy-17α-trifluormethyl-androst-4-en-3-on
17β-Hydroxy-17α-trifluormethyl-estr-4-en-3-on,
17β-Hydroxy-17α-trifluormethyl-estr-4,9-dien-3-on,
17β-Hydroxy-17α-trifluormethyl-estr-4,9,11-trien-3-on,
13-Ethyl-17β-hydroxy-17α-triffuormethyl-gon-4-en-3-on,
13-Ethyl-17β-hydroxy-17α-trifluormethyl-gon-4,9-dien-3-on und
13-Ethyl-17β-hydroxy-17α-trifluormethyl-gon-4,9,11-trien-3-on.
zur Herstellung von Arzneimitteln zur Fertilitätskontrolle beim Mann, zur Hormon-Replacement-Therapie (HRT) bei Mann und Frau oder zur Behandlung hormonell bedingter Erkrankungen bei Mann und Frau, wie beispielsweise Endometriose, Mammacarcinom oder Hypogonadismus.

## Claims

1. 17α-Fluoroalkyl steroids of general formula (I) 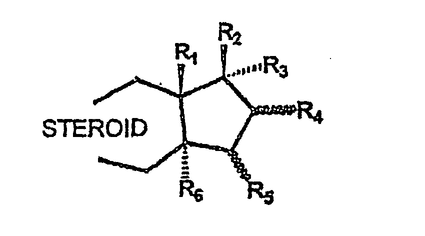 in which R¹ stands for a C₁₋₄-alkyl group,
R² stands for a hydroxy group, a group OC(O)-R²⁰ or OR²¹, wherein R²⁰ and R²¹ mean a C₁₋₁₂-alkyl group, a C₃₋₈-cycloalkyl group, an aryl group which has 6 to 15 carbon atoms and can be substituted or an aryl-C₁₋₄-alkyl group which together has 7 to 15 carbon atoms and can be substituted at the aryl radical:
R³ stands for a radical of formula CₙF₂ₙ₊₁, wherein n=1, 2, 3, 4, 5 or 6,
R⁴ and R⁵ in each case stand for a hydrogen atom, together for a double bond or a methylene bridge,
R⁵ and R⁶ each stand for a hydrogen atom, together for a double bond or a methylene bridge, STEROID stands for a steroidal ABC-ring system of partial formulas A, B, C, D, E and F: 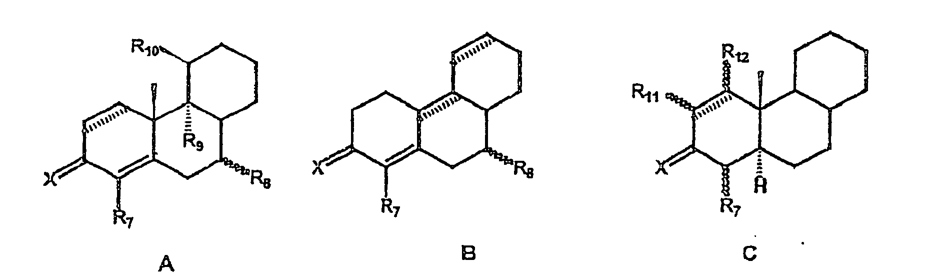 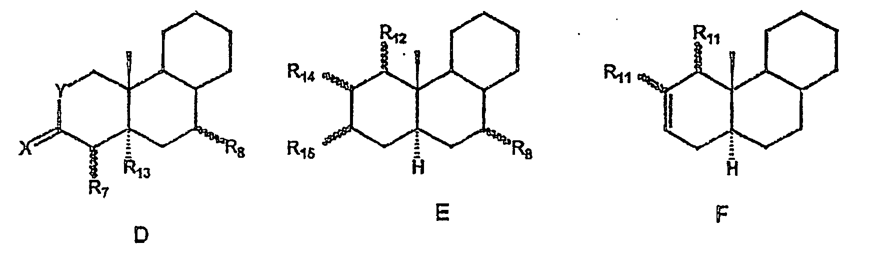 wherein an additional double bond can be found in A and C in 1,2-position, and one or two additional double bonds can be found in B in 8,9-position and 11,12-position,
R⁷ means a hydrogen atom, a halogen atom, a hydroxyl group or a trifluoromethyl group,
X means an oxygen atom, or two hydrogen atoms,
R⁸ means a hydrogen atom, a methyl or ethyl group,
R⁹ means a hydrogen atom or a halogen atom or together with R¹⁰ stands for a double bond,
R¹⁰ means a hydrogen atom, a hydroxyl group, a methyl or ethyl group or together with R⁹ stands for a double bond,
R¹¹ means a hydrogen atom, a C₁₋₄-alkyl group, a nitrile group, a hydroxy-methylene group or a formyl group,
R¹² means a hydrogen atom, a C₁₋₄-alkyl group or a nitrile group,
R¹¹ and R¹², in addition to the above-mentioned meanings, together mean a methylene bridge,
R¹³ means a hydrogen atom or together with R⁷ means a double bond,
R¹⁹ and R¹⁵ together stand for a double bond, an oxirane ring, a thiirane ring, a [2,3c]oxadiazole ring, a [3,2c]isoxazole ring or a [3,2c]pyrazole ring,
Y stands for an oxygen or nitrogen atom,
and the wavy lines at R⁷, R⁸, R¹¹, R¹², R¹³, R¹⁴ and R¹⁵ mean that these substituents can be in α- or β-position, and the following compounds are excluded:
17β-Hydroxy-17α-trifluoromethyl-androst-4-en-3-one
17β-Hydroxy-17α-trifluoromethyl-estr-4-en-3-one,
17β-Hydroxy-17α-trifluoromethyl-estra-4,9-dien-3-one,
17β-Hydroxy-17α-trifluoromethyl-estra-4,9,11-trien-3-one,
13-Ethyl-17β-hydroxy-17α-trifluoromethyl-gon-4-en-3-one,
13-Ethyl-17β-hydroxy-17α-trifluoromethyl-gona-4,9-dien-3-one and
13-Ethyl-17β-hydroxy-17α-trifluoromethyl-gona-4,9,11-trien-3-one.

2. 17α-Fluoroalkyl steroids according to claim 1, wherein STEROID stands for a steroidal ring system of partial formula A.

3. 17α-Fluoroalkyl steroids according to claim 1, wherein STEROID stands for a steroidal ring system of partial formula B.

4. 17α-Fluoroalkyl steroids according to claim 1, wherein STEROID stands for a steroidal ring system of partial formula C.

5. 17α-Fluoroalkyl steroids according to claim 1, wherein STEROID stands for a steroidal ring system of partial formula D.

6. 17α-Fluoroalkyl steroids according to claim 1, wherein STEROID stands for a steroidal ring system of partial formula E.

7. 17α-Fluoroalkyl steroids according to claim 1, wherein STEROID stands for a steroidal ring system of partial formula F.

8. 17α-Fluoroalkyl steroids according to one of claims 1 to 7, wherein R¹ represents a methyl group.

9. 17α-Fluoroalkyl steroids according to one of claims 1 to 8, wherein R² means a hydroxy group, a formyloxy group, an acetyloxy group, a propionyloxy group, a butyryloxy group, a [(trans-4-butylcyclohexyl)carbonyl]oxy group, a phenylpropionyloxy group, an iso-butyryloxy group, a heptanyloxy group or an undecanyloxy group.

10. 17α-Fluoroalkyl steroids according to one of claims 1 to 9, wherein R³ represents a trifluoromethyl group or a pentafluoroethyl group.

11. 17α-Fluoroalkyl steroids according to one of claims 1 to 3 as well as 8 to 10, wherein R₈ is a methyl group.

12. 17α-Fluoroalkyl steroids according to one of claims 1 to 5 as well as 8 to 11, wherein R₇ represents a fluorine, chlorine or bromine atom, or a trifluoromethyl or hydroxy group.

13. 17α-Fluoroalkyl steroids according to one of claims 1, 2 as well as 8 to 11, wherein R¹⁰ represents a hydroxy group.

14. 17α-Fluoroalkyl steroids according to one of claims 1, 2 as well as 8 to 12, wherein R⁹ is a fluorine atom.

15. 17α-Fluoroalkyl steroids according to one of claims 1, 4, 7 and 12, wherein R¹¹ represents a hydroxymethylene group or a formyl group.

16. 17α-Fluoroalkyl steroids according to one of claims 1, 5, 11 and 12, wherein Y is an oxygen atom.

17. 17α-Fluoroalkyl steroids according to claim 1, namely
17β-Hydroxy-17α-trifluoromethyl-7α-methyl-androst-4-en-3-one,
17β,4-Dihydroxy-17α-trifluoromethyl-androst-4-en-3-one,
17β-Hydroxy-17α-trifluoromethyl-4-chloro-androst-4-en-3-one,
17β-Hydroxy-17α-trifluoromethyl-4-bromo-androst-4-en-3-one,
17β-Hydroxy-17α,4-bis(trifluoromethyl)-androst-4-en-3-one,
17β,11β-Dihydroxy-17α-trifluoromethyl-androst-4-en-3-one,
17β, 11β-Dihydroxy-17α-trifluoromethyl-9α-fluoro-androst-4-en-3-one,
17β-Hydroxy-17α-trifluoromethyl-androsta-1,4-dien-3-one,
17β-Hydroxy-17α-trifluoromethyl-4-chloro-androsta-1,4-dien-3-one,
17β,4-Dihydroxy-17α-trifluoromethyl-androsta-1,4-dien-3-one,
17β-Hydroxy-17α-trifluoromethyl-7α-methyl-androsta-1,4-dien-3-one,
17β-Hydroxy-17α-trifluoromethyl-7α-methyl-4-chloro-androsta-1,4-dien-3-one,
17β-Hydroxy-17α-pentafluoroethyl-androst-4-en-3-one,
17β-Hydroxy-17α-pentafluoroethyl-7α-methyl-androst-4-en-3-one,
17β,4-Dihydroxy-17α-pentafluoroethyl-androst-4-en-3-one,
17β-Hydroxy-17α-pentafluoroethyl-4-chloro-androst-4-en-3-one,
17β-Hydroxy-17α-pentafluoroethyl-4-bromo-androst-4-en-3-one,
17β-Hydroxy-17α-pentafluoroethyl-4-trifluoromethyl-androst-4-en-3-one,
17β, 11β-Dihydroxy-17α-pentafluoroethyl-androst-4-en-3-one,
17β, 11β-Dihydroxy-17α-pentafluoroethyl-9α-fluoro-androst-4-en-3-one,
17β-Hydroxy-17α-pentafluoroethyl-androsta-1,4-dien-3-one,
17β-Hydroxy-17α-pentafluoroethyl-4-chloro-androsta-1,4-dien-3-one,
17β, 4-Dihydroxy-17α-pentafluoroethyl-androsta-1,4-dien-3-one,
17β-Hydroxy-17α-pentafluoroethyl-4-trifluoromethyl-androsta-1,4-dien-3-one,
17β-Hydroxy-17α-pentafluoroethyl-7α-methyl-androsta-1,4-dien-3-one,
17β-Hydroxy-17α-pentafluoroethyl-7α-methyl-4-chloro-androsta-1,4-dien-3-one,
17β-Hydroxy-17α-trifluoromethyl-7α-methyl-estr-4-en-3-one,
17α,4-Dihydroxy-17α-trifluoromethyl-estr-4-en-3-one,
17β-Hydroxy-17α-trifluoromethyl-4-chloro-estr-4-en-3-one,
17β-Hydroxy-17α-trifluoromethyl-4-bromo-estr-4-en-3-one,
17β-Hydroxy-17α,4-bis(trifluoromethyl)-estr-4-en-3-one,
17β-Hydroxy-17α-trifluoromethyl-7α-methyl-estra-4,9-dien-3-one,
17β-Hydroxy-17α-trifluoromethyl-7α-methyl-estra-4,9,11-trien-3-one,
17β-Hydroxy-17α-pentafluoroethyl-estr-4-en-3-one,
17β-Hydroxy-17α-pentafluoroethyl-7α-methyl-estr-4-en-3-one,
17β,4-Dihydroxy-17α-pentafluoroethyl-estr-4-en-3-one,
17β-Hydroxy-17α-pentafluoroethyl-4-chloro-estr-4-en-3-one,
17β-Hydroxy-17α-pentafluoroethyl-4-bromo-estr-4-en-3-one,
17β-Hydroxy-17α-pentafluoroethyl-4-trifluoromethyl-estr-4-en-3-one,
17β-Hydroxy-17α-pentafluoroethyl-estra-4,9-dien-3-one,
17β-Hydroxy-17α-pentafluoroethyl-estra-4,9,11-trien-3-one,
17β-Hydroxy-17α-pentafluoroethyl-7α-methyl-estra-4,9-dien-3-one,
17β-Hydroxy-17α-pentafluoroethyl-7α-methyl-estra-4,9,11-trien-3-one,
13-Ethyl-17β-hydroxy-17α-trifluoromethyl-4-chloro-gon-4-en-3-one,
13-Ethyl-17β,4-dihydroxy-17α-trifluoromethyl-gon-4-en-3-one,
13-Ethyl-17β-hydroxy-17α-trifluoromethyl-7α-methyl-gon-4-en-3-one,
13-Ethyl-17β-hydroxy-17α-trifluoromethyl-7α-methyl-gona-4,9-dien-3-one,
13-Ethyl-17β-hydroxy-17α-trifluoromethyl-7α-methyl-gona-4,9,11-trien-3-one,
13-Ethyl-17β-hydroxy-17α-pentafluoroethyl-gon-4-en-3-one,
13-Ethyl-17β-hydroxy-17α-pentafluoroethyl-7α-methyl-gon-4-en-3-one,
13-Ethyl-17β,4-dihydroxy-17α-pentafluoroethyl-gon-4-en-3-one,
13-Ethyl-17β-hydroxy-17α-pentafluoroethyl-4-chloro-gon-4-en-3-one,
13-Ethyl-17β-hydroxy-17α-pentafluoroethyl-4-bromo-gon-4-en-3-one,
13-Ethyl-17β-hydroxy-17α-pentafluoroethyl-4-trifluoromethyl-gon-4-en-3-one,
13-Ethyl-17β-hydroxy-17α-pentafluoroethyl-gona-4,9-dien-3-one,
13-Ethyl-17β-hydroxy-17α-pentafluoroethyl-gona-4,9,11-trien-3-one,
13-Ethyl-17β-hydroxy-17α-pentafluoroethyl-7α-methyl-gona-4,9-dien-3-one,
13-Ethyl-17β-hydroxy-17α-pentafluoroethyl-7α-methyl-gona-4,9,11-trien-3-one,
17β-Hydroxy-17α-trifluoromethyl-5α-androstan-3-one,
17β-Hydroxy-17α-pentafluoroethyl-5α-androstan-3-one,
17β-Hydroxy-17α-trifluoromethyl-7α-methyl-5α-androstan-3-one,
17β-Hydroxy-17α-pentafluoroethyl-7α-methyl-5α-androstan-3-one,
17β-Hydroxy-17α-trifluoromethyl-2-hydroxymethylene-5α-androstan-3-one,
17β-Hydroxy-17α-pentafluoroethyl-2-hydroxymethylene-5α-androstan-3-one,
17β-Hydroxy-17α-trifluoromethyl-2α-methyl-5α-androstan-3-one,
17β-Hydroxy-17α-pentafluoroethyl-2α-methyl-5α-androstan-3-one,
17β-Hydroxy-17α-trifluoromethyl-1α-methyl-5α-androstan-3-one,
17β-Hydroxy-17α-pentafluoroethyl-1α-methyl-5α-androstan-3-one,
17β-Hydroxy-17α-trifluoromethyl-5α-androst-2-ene,
17β-Hydroxy-17α-pentafluoroethyl-5α-androst-2-ene,
17β-Hydroxy-17α-trifluoromethyl-2-methyl-5α-androst-2-ene,
17β-Hydroxy-17α-pentafluoroethyl-2-methyl-5α-androst-2-ene,
17β-Hydroxy-17α-trifluoromethyl-2-cyano-5α-androst-2-ene,
17β-Hydroxy-17α-pentafluoroethyl-2-cyano-5α-androst-2-ene,
17β-Hydroxy-17α-trifluoromethyl-2-formyl-5α-androst-2-ene,
17β-Hydroxy-17α-pentafluoroethyl-2-formyl-5α-androst-2-ene,
17β-Hydroxy-17α-trifluoromethyl-[2,3c]oxadiazole-5α-androstane,
17β-Hydroxy-17α-pentafluoroethyl-[2,3c]oxadiazole-5α-androstane,
17β-Hydroxy-17α-trifluoromethyl-[3,2c]isoxazole-5α-androstane,
17β-Hydroxy-17α-pentafluoroethyl-[3,2c]isoxazole-5α-androstane,
17β-Hvdroxy-17α-trifluoromethyl-[3,2c]pyrazole-5α-androstane,
17β-Hydroxy-17α-pentafluoroethyl-[3,2c]pyrazole-5α-androstane,
17β-Hydroxy-17α-trifluoromethyl-2β,3β-epithio-5a-androstane,
17β-Hydroxy-17α-pentafluoroethyl-2β,3β-epithio-5α-androstane,
17β-Hydroxy-17α-trifluoromethyl-2α,3α-epithio-5α-androstane,
17β-Hydroxy-17α-pentafluoroethyl-2α,3α-epithio-5α-androstane,
17β-Hydroxy-17α-trifluoromethyl-2-oxa-5α-androstan-3-one,
17β-Hydroxy-17α-pentafluoroethyl-2-oxa-5α-androstan-3-one,
17β-Hydroxy-17α-trifluoromethyl-5α-androst-1-en-3-one,
17β-Hydroxy-17α-pentafluoroethyl-5α-androst-1-en-3-one,
17β-Hydroxy-17α-trifluoromethyl-1-methyl-5α-androst-1-en-3-one,
17β-Hydroxy-17α-pentafluoroethyl-1-methyl-5α-androst-1-en-3-one,
17β-Hydroxy-17α-trifluoromethyl-2-methyl-5α-androst-1-en-3-one and
17β-Hydroxy-17α-pentafluoroethyl-2-methyl-5α-androst-1-en-3-one.

18. Process for the production of 17α-fluoroalkyl steroids of general formula (I) according to claim 1, **characterized in that** compounds of general formula (II) 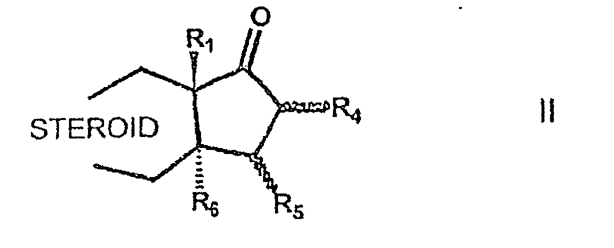 in which R¹, R⁴, R⁵, R⁶ and STEROID, which have the meanings given in claim 1, are reacted in the presence of fluoride with perfluoroalkyltrialkylsilanes, (Alk) ₃SiCₙF₂ₙ₊₁, or with fluoroalkyl lithium, LiCₙF₂ₙ₊₁, or fluoroalkyl Grignard reagents, ZMgCₙF₂ₙ₊₁, wherein n=1, 2, 3, 4, 5 or 6, Z is a chlorine, bromine or iodine atom, and Alk is a C₁₋₄-alkyl radical.

19. Pharmaceutical compositions containing at least one 17α-fluoroalkyl steroid of general formula (I) according to one of claims 1 to 17, optionally together with pharmaceutically compatible adjuvants and vehicles.

20. 17α-Fluoroalkyl steroids of formula (I) according to one of claims 1 to 17 for use as therapeutic active ingredients.

21. Use of 17α-fluoroalkyl steroids of general formula (I) 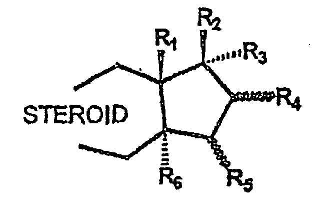 in which R¹ stands for a C₁₋₄-alkyl group,
R² stands for a hydroxy group, a group OC(O)-R²⁰ or OR²¹, wherein R²⁰ and R²¹ mean a C₁₋₁₂-alkyl group, a C₃₋₈-cycloalkyl group, an aryl group which has 6 to 15 carbon atoms and can be substituted or an aryl-C₁₋₄-alkyl group which together has 7 to 15 carbon atoms and can be substituted at the aryl radical:
R³ stands for a radical of formula CₙF₂ₙ₊₁, wherein n=1, 2, 3, 4, 5 or 6,
R⁴ and R⁵ in each case stand for a hydrogen atom, together for a double bond or a methylene bridge,
R⁵ and R⁶ each stand for a hydrogen atom, together for a double bond or a methylene bridge, STEROID stands for a steroidal ABC-ring system of partial formulas A, B, C, D, E and F: 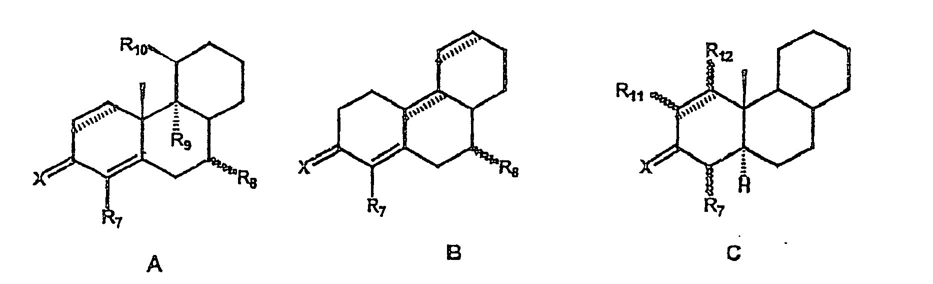 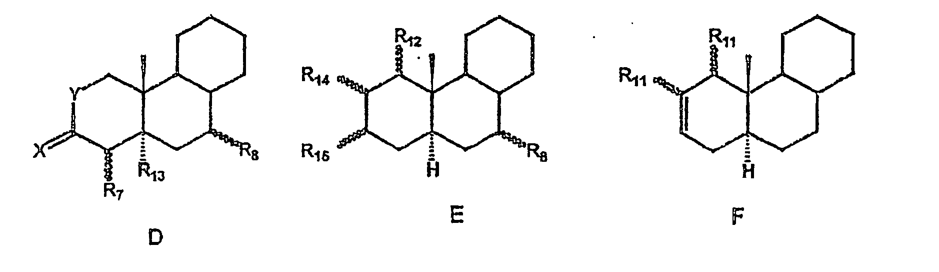 wherein an additional double bond can be found in A and C in 1,2-position, and one or two additional double bonds can be found in B in 8,9-position and 11,12-position,
R⁷ means a hydrogen atom, a halogen atom, a hydroxyl group or a trifluoromethyl group,
X means an oxygen atom, or two hydrogen atoms,
R⁸ means a hydrogen atom, a methyl or ethyl group,
R⁹ means a hydrogen atom or a halogen atom or together with R¹⁰ stands for a double bond,
R¹⁰ means a hydrogen atom, a hydroxyl group, a methyl or ethyl group or together with R⁹ stands for a double bond,
R¹¹ means a hydrogen atom, a C₁₋₄-alkyl group, a nitrile group, a hydroxy-methylene group or a formyl group,
R¹² means a hydrogen atom, a C₁₋₄-alkyl group or a nitrile group,
R¹¹ and R¹², in addition to the above-mentioned meanings, together mean a methylene bridge,
R¹³ means a hydrogen atom or together with R⁷ means a double bond,
R¹⁴ and R¹⁵ together stand for a double bond, an oxirane ring, a thiirane ring, a [2,3c]oxadiazole ring, a [3,2c]isoxazole ring or a [3,2c]pyrazole ring,
Y stands for an oxygen or nitrogen atom,
and the wavy lines at R⁷, R⁸, R¹¹, R¹², R¹³, R¹⁴ and R¹⁵ mean that these substituents can be in α- or β-position, and the following compounds are excluded:
17β-Hydroxy-17α-trifluoromethyl-androst-4-en-3-one
17β-Hydroxy-17α-trifluoromethyl-estr-4-en-3-one,
17β-Hydroxy-17α-trifluoromethyl-estra-4,9-dien-3-one,
17β-Hydroxy-17α-trifluoromethyl-estra-4,9,11-trien-3-one,
13-Ethyl-17β-hydroxy-17α-trifluoromethyl-gon-4-en-3-one,
13-Ethyl-17β-hydroxy-17α-trifluoromethyl-gona-4,9-dien-3-one and
13-Ethyl-17β-hydroxy-17α-trifluoromethyl-gona-4,9,11-trien-3-one,
for birth control in men, for hormone replacement therapy (HRT) in men and women or for treatment of hormonally induced diseases in men and women, such as, for example, endometriosis, breast cancer or hypogonadism.

## Revendications

1. 17α-fluoralkylstéroïdes de formule générale (I) 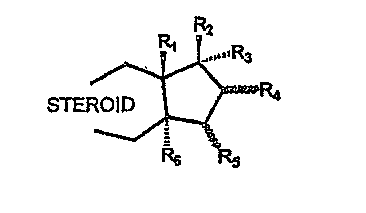 dans laquelle R¹ est un groupe alkyle en C₁₋₄,
R² est un groupe hydroxy, un groupe O-C (O) -R²⁰ ou OR²¹, où R²⁰ et R²¹ représentent chacun un groupe alkyle en C₁₋₁₂, un groupe cycloalkyle en C₃₋₈, un groupe aryle ayant 6 à 15 atomes de carbone, qui peut être substitué, ou un groupe aryl (alkyle en C₁₋₄) qui comprend en tout 7 à 15 atomes de carbone et peut être substitué sur le radical aryle,
R³ est un radical de formule CₙF₂ₙ₊₁, dans laquelle n = 1, 2, 3, 4, 5 ou 6,
R⁴ et R⁵ représentent chacun un atome d'hydrogène, ou forment ensemble une double liaison ou un pont méthylène,
R⁵ et R⁶ représentent chacun un atome d'hydrogène, ou forment ensemble une double liaison ou un pont méthylène,
STEROID désigne un système cyclique de type stéroïde ABC, ayant les formules partielles A, B, C, D, E et F, 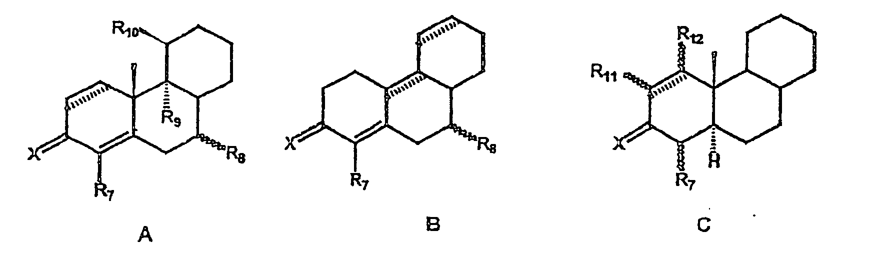 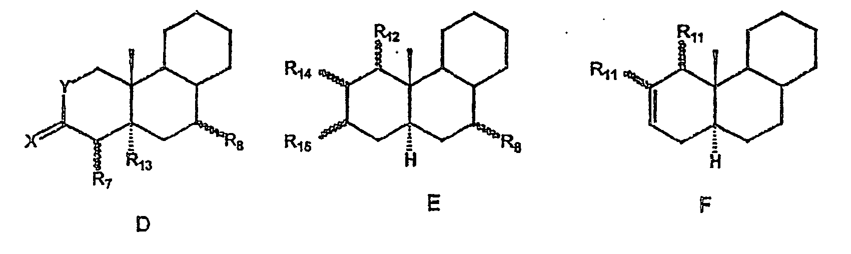 où on peut avoir en A et en C en position 1,2 une double liaison supplémentaire, et en B, en position 8,9 et en position 11,12, une ou deux doubles liaisons supplémentaires,
R⁷ est un atome d'hydrogène, un atome d'halogène, un groupe hydroxyle ou un groupe trifluorométhyle,
X est un atome d'hydrogène ou représente deux atomes d'hydrogène,
R⁸ est un atome d'hydrogène, un groupe méthyle ou éthyle,
R⁹ est un atome d'hydrogène ou un atome d'halogène, ou ensemble avec R¹⁰ forme une double liaison,
R¹⁰ est un atome d'hydrogène, un groupe hydroxyle, un groupe méthyle ou éthyle, ou ensemble avec R⁹ forme une double liaison,
R¹¹ est un atome d'hydrogène, un groupe alkyle en C₁₋₄, un groupe nitrile, un groupe hydroxyméthylène ou formyle,
R¹² est un atome d'hydrogène, un groupe alkyle en C₁₋₄ ou un groupe nitrile,
R¹¹ et R¹², outre les significations mentionnées ci-dessus, représentent ensemble un pont méthylène,
R¹³ est un atome d'hydrogène, ou ensemble avec R⁷ forme une double liaison,
R¹⁴ et R¹⁵ forment ensemble une double liaison, un noyau oxiranne, un noyau thiiranne, un noyau [2,3c]oxadiazole, un noyau [3,2c]isoxazole ou un noyau [3,2c]pyrazole,
Y est un atome d'oxygène ou d'azote,
où les lignes ondulées, au niveau de R⁷, R⁸, R¹¹, R¹², R¹³, R¹⁴ et R¹⁵, indiquent que ces substituants peuvent être en position α ou β, à l'exception des composés suivants :
17β-hydroxy-17α-trifluorométhyl-androst-4-ène-3-one,
17β-hydroxy-17α-trifluorométhyl-oestr-4-ène-3-one,
17β-hydroxy-17α-trifluorométhyl-oestr-4,9-diène-3-one,
17β-hydroxy-17α-trifluorométhyl-oestr-4,9,11-triène-3-one,
13-éthyl-17β-hydroxy-17α-trifluorométhyl-gone-4-ène-3-one,
13-éthyl-17β-hydroxy-17α-trifluorométhyl-gone-4,9-diène-3-one et
13-éthyl-17β-hydroxy-17α-trifluorométhyl-gone-4,9,11-triène-3-one.

2. 17α-fluoralkylstéroïdes selon la revendication 1, dans lesquels STEROID représente un système cyclique de type stéroïde ayant la formule partielle A.

3. 17α-fluoralkylstéroïdes selon la revendication 1, dans lesquels STEROID représente un système cyclique de type stéroïde ayant la formule partielle B.

4. 17α-fluoralkylstéroïdes selon la revendication 1, dans lesquels STEROID représente un système cyclique de type stéroïde ayant la formule partielle C.

5. 17α-fluoralkylstéroïdes selon la revendication 1, dans lesquels STEROID représente un système cyclique de type stéroïde ayant la formule partielle D.

6. 17α-fluoralkylstéroïdes selon la revendication 1, dans lesquels STEROID représente un système cyclique de type stéroïde ayant la formule partielle E.

7. 17α-fluoralkylstéroïdes selon la revendication 1, dans lesquels STEROID représente un système cyclique de type stéroïde ayant la formule partielle F.

8. 17α-fluoralkylstéroïdes selon l'une des revendications 1 à 7, dans lesquels R¹ est un groupe méthyle.

9. 17α-fluoralkylstéroïdes selon l'une des revendications 1 à 8, dans lesquels R² est un groupe hydroxy, un groupe formyloxy, un groupe acétyloxy, un groupe propionyloxy, un groupe butyryloxy, un groupe [(trans-4-butylcyclohexyl)carbonyl]oxy, un groupe phénylpropionyloxy, un groupe isobutyryloxy, un groupe heptanyloxy ou un groupe undécanyloxy.

10. 17α-fluoralkylstéroïdes selon l'une des revendications 1 à 9, dans lesquels R³ est un groupe trifluorométhyle ou pentafluoréthyle.

11. 17α-fluoralkylstéroïdes selon l'une des revendications 1 à 3, ainsi que 8 à 10, dans lesquels R⁸ est un groupe méthyle.

12. 17α-fluoralkylstéroïdes selon l'une des revendications 1 à 5, ainsi que 8 à 11, dans lesquels R⁷ est un atome de fluor, de chlore ou de brome, ou un groupe trifluorométhyle ou hydroxy.

13. 17α-fluoralkylstéroïdes selon l'une des revendications 1, 2, ainsi que 8 à 11, dans lesquels R¹⁰ est un groupe hydroxy.

14. 17α-fluoralkylstéroïdes selon l'une des revendications 1, 2, ainsi que 8 à 12, dans lesquels R⁹ est un atome de fluor.

15. 17α-fluoralkylstéroïdes selon l'une des revendications 1, 4, 7 et 12, dans lesquels R¹¹ est un groupe hydroxyméthylène ou formyle.

16. 17α-fluoralkylstéroïdes selon l'une des revendications 1, 5, 11 et 12, dans lesquels Y est un atome d'oxygène.

17. 17α-fluoralkylstéroïdes selon la revendication 1, et plus précisément les composés suivants :
17β-hydroxy-17α-trifluorométhyl-7α-méthyl-androst-4-ène-3-one,
17β,4-dihydroxy-17α-trifluorométhyl-androst-4-ène-3-one,
17β-hydroxy-17α-trifluorométhyl-4-chloro-androst-4-ène-3-one,
17β-hydroxy-17α-trifluorométhyl-4-bromo-androst-4-ène-3-one,
17β-hydroxy-17α,4-bis(trifluorométhyl)-androst-4-ène-3-one,
17β,11β-dihydroxy-17α-trifluorométhyl-androst-4-ène-3-one,
17β, 11β-dihydroxy-17α-trifluorométhyl-9α-fluoro-androst-4-ène-3-one,
17β-hydroxy-17α-trifluorométhyl-androst-1,4-diène-3-one,
17β-hydroxy-17α-trifluorométhyl-4-chloro-androst-1,4-diène-3-one,
17β,4-dihydroxy-17α-trifluorométhyl-androst-1,4-diène-3-one,
17β-hydroxy-17α-trifluorométhyl-7α-méthyl-androst-1,4-diène-3-one,
17β-hydroxy-17α-trifluorométhyl-7α-méthyl-4-chloro-androst-1,4-diène-3-one,
17β-hydroxy-17α-pentafluoréthyl-androst-4-ène-3-one,
17β-hydroxy-17α-pentafluoréthyl-7α-méthyl-androst-4-ène-3-one,
17β, 4-dihydroxy-17α-pentafluoréthyl-androst-4-ène-3-one,
17β-hydroxy-17α-pentafluoréthyl-4-chloro-androst-4-ène-3-one,
17β-hydroxy-17α-pentafluoréthyl-4-bromo-androst-4-ène-3-one,
17β-hydroxy-17α-pentafluoréthyl-4-trifluorométhyl-androst-4-ène-3-one,
17β, 11β-dihydroxy-17α-pentafluoréthyl-androst-4-ène-3-one,
17β, 11β-dihydroxy-17α-pentafluoréthyl-9α-fluoro-androst-4-ène-3-one,
17β-hydroxy-17α-pentafluoréthyl-androst-1,4-diène-3-one,
17β-hydroxy-17α-pentafluoréthyl-4-chloro-androst-1,4-diène-3-one,
17β,4-dihydroxy-17α-pentafluoréthyl-androst-1,4-diène-3-one,
17β-hydroxy-17α-pentafluoréthyl-4-trifluorométhyl-androst-1,4-diène-3-one,
17β-hydroxy-17α-pentafluoréthyl-7α-méthyl-androst-1,4-diène-3-one,
17β-hydroxy-17α-pentafluoréthyl-7α-méthyl-4-chloro-androst-1,4-diène-3-one,
17β-hydroxy-17α-trifluorométhyl-7α-méthyl-oestr-4-ène-3-one,
17β,4-dihydroxy-17α-trifluorométhyl-oestr-4-ène-3-one,
17β-hydroxy-17α-trifluorométhyl-4-chloro-oestr-4-ène-3-one,
17β-hydroxy-17α-trifluorométhyl-4-bromo-oestr-4-ène-3-one,
17β-hydroxy-17α,4-bis(trifluorométhyl)-oestr-4-ène-3-one,
17β-hydroxy-17α-trifluorométhyl-7α-méthyl-oestr-4,9-diène-3-one,
17β-hydroxy-17α-trifluorométhyl-7α-méthyl-oestr-4,9,11-triène-3-one,
17β-hydroxy-17α-pentafluoréthyl-oestr-4-ène-3-one,
17β-hydroxy-17α-pentafluoréthyl-7α-méthyl-oestr-4-ène-3-one,
17β,4-dihydroxy-17α-pentafluoréthyl-oestr-4-ène-3-one,
17β-hydroxy-17α-pentafluoréthyl-4-chloro-oestr-4-ène-3-one,
17β-hydroxy-17α-pentafluoréthyl-4-bromo-oestr-4-ène-3-one,
17β-hydroxy-17α-pentafluoréthyl-4-trifluorométhyl-oestr-4-ène-3-one,
17β-hydroxy-17α-pentafluoréthyl-oestr-4,9-diène-3-one,
17β-hydroxy-17α-pentafluoréthyl-oestr-4,9,11-triène-3-one,
17β-hydroxy-17α-pentafluoréthyl-7α-méthyl-oestr-4,9-diène-3-one,
17β-hydroxy-17α-pentafluoréthyl-7α-méthyl-oestr-4,9,11-triène-3-one,
13-éthyl-17β-hydroxy-17α-trifluorométhyl-4-chloro-gone-4-ène-3-one,
13-éthyl-17β,4-dihydroxy-17α-trifluorométhyl-gone-4-ène-3-one,
13-éthyl-17β-hydroxy-17α-trifluorométhyl-7α-méthyl-gone-4-ène-3-one,
13-éthyl-17β-hydroxy-17α-trifluorométhyl-7α-méthyl-gone-4,9-diène-3-one,
13-éthyl-17β-hydroxy-17α-trifluorométhyl-7α-méthyl-gone-4,9,11-triène-3-one,
13-éthyl-17β-hydroxy-17α-pentafluoréthyl-gone-4-ène-3-one,
13-éthyl-17β-hydroxy-17α-pentafluoréthyl-7α-méthyl-gone-4-ène-3-one,
13-éthyl 17β,4-dihydroxy-17α-pentafluoréthyl-gone-4-ène-3-one,
13-éthyl-17β-hydroxy-17α-pentafluoréthyl-4-chloro-gone-4-ène-3-one,
13-éthyl-17β-hydroxy-17α-pentafluoréthyl-4-bromo-gone-4-ène-3-one,
13-éthyl-17β-hydroxy-17α-pentafluoréthyl-4-trifluorométhyl-gone-4-ène-3-one,
13-éthyl-17β-hydroxy-17α-pentafluoréthyl-gone-4,9-diène-3-one,
13-éthyl-17β-hydroxy-17α-pentafluoréthyl-gone-4,9,11-triène-3-one,
13-éthyl-17β-hydroxy-17α-pentafluoréthyl-7α-méthyl-gone-4,9-diène-3-one,
13-éthyl-17β-hydroxy-17α-pentafluoréthyl-7α-méthyl-gone-4,9,11-triène-3-one,
17β-hydroxy-17α-trifluorométhyl-5α-androstane-3-one,
17β-hydroxy-17α-pentafluoréthyl-5α-androstane-3-one,
17β-hydroxy-17α-trifluorométhyl-7α-méthyl-5α-androstane-3-one,
17β-hydroxy-17α-pentafluoréthyl-7α-méthyl-5α-androstane-3-one,
17β-hydroxy-17α-trifluorométhyl-2-hydroxyméthylène-5α-androstane-3-one,
17β-hydroxy-17α-pentafluoréthyl-2-hydroxyméthylène-5α-androstane-3-one,
17β-hydroxy-17α-trifluorométhyl-2α-méthyl-5α-androstane-3-one,
17β-hydroxy-17α-pentafluoréthyl-2α-méthyl-5α-androstane-3-one,
17β-hydroxy-17α-trifluorométhyl-1α-méthyl-5α-androstane-3-one,
17β-hydroxy-17α-pentafluoréthyl-1α-méthyl-5α-androstane-3-one,
17β-hydroxy-17α-trifluorométhyl-5α-androst-2-ène,
17β-hydroxy-17α-pentafluoréthyl-5α-androst-2-ène,
17β-hydroxy-17α-trifluorométhyl-2-méthyl-5α-androst-2-ène,
17β-hydroxy-17α-pentafluoréthyl-2-méthyl-5α-androst-2-ène,
17β-hydroxy-17α-trifluorométhyl-2-cyano-5α-androst-2-ène,
17β-hydroxy-17α-pentafluoréthyl-2-cyano-5α-androst-2-ène,
17β-hydroxy-17α-trifluorométhyl-2-formyl-5α-androst-2-ène,
17β-hydroxy-17α-pentafluoréthyl-2-formyl-5α-androst-2-ène,
17β-hydroxy-17α-trifluorométhyl-[2,3c]oxadiazole-5α-androstane,
17β-hydroxy-17α-pentafluoréthyl-[2,3c]oxadiazole-5α-androstane,
17β-hydroxy-17α-trifluorométhyl-[3,2c]isoxazole-5α-androstane,
17β-hydroxy-17α-pentafluoréthyl-[3,2c]isoxazole-5α-androstane,
17β-hydroxy-17α-trifluorométhyl-[3,2c]pyrazole-5α-androstane,
17β-hydroxy-17α-pentafluoréthyl-[3,2c]pyrazole-5α-androstane,
17β-hydroxy-17α-trifluorométhyl-2β,3β-épithio-5α-androstane,
17β-hydroxy-17α-pentafluoréthyl-2β,3β-épithio-5α-androstane,
17β-hydroxy-17α-trifluorométhyl-2α,3α-épithio-5α-androstane,
17β-hydroxy-17α-pentafluoréthyl-2α,3α-épithio-5α-androstane,
17β-hydroxy-17α-trifluorométhyl-2-oxa-5α-androstane-3-one,
17β-hydroxy-17α-pentafluoréthyl-2-oxa-5α-androstane-3-one,
17β-hydroxy-17α-trifluorométhyl-5α-androst-1-ène-3-one,
17β-hydroxy-17α-pentafluoréthyl-5α-androst-1-ène-3-one,
17β-hydroxy-17α-trifluorométhyl-1-méthyl-5α-androst-1-ène-3-one,
17β-hydroxy-17α-pentafluoréthyl-1-méthyl-5α-androst-1-ène-3-one,
17β-hydroxy-17α-trifluorométhyl-2-méthyl-5α-androst-1-ène-3-one, et
17β-hydroxy-17α-pentafluoréthyl-2-méthyl-5α-androst-1-ène-3-one.

18. Procédé de préparation de 17α-fluoralkylstéroïdes de formule (I) selon la revendication 1, **caractérisé en ce qu'**on fait réagir des composés de formule générale (II) 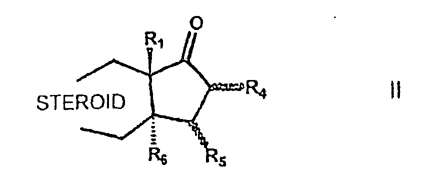 dans laquelle R¹, R⁴ R⁵ R⁶ et STEROID ont les significations données dans la revendication 1, en présence d'un fluorure, avec des perfluoralkyltrialkylsilanes (Alk)₃SiCₙF₂ₙ₊₁ ou avec un fluoralkyllithium LiCₙF₂ₙ₊₁ ou des réactifs de Grignard fluoralkylés ZMgCₙF₂ₙ₊₁, où n = 1, 2, 3, 4, 5 ou 6, Z est un atome de chlore, de brome ou d'iode, et Alk désigne un radical alkyle en C₁₋₄.

19. Compositions pharmaceutiques qui contiennent au moins un 17α-fluoralkylstéroïde de formule générale (I) selon l'une des revendications 1 à 17, éventuellement en même temps que des adjuvants et excipients acceptables d'un point de vue pharmaceutique.

20. 17α-fluoralkylstéroïdes de formule (I) selon l'une des revendications 1 à 17, pour utilisation en tant que principes actifs thérapeutiques.

21. Utilisation des 17α-fluoralkylstéroïdes de formule générale (I) 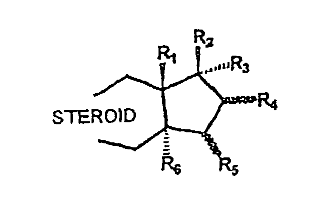 dans laquelle R¹ est un groupe alkyle en C₁₋₄,
R² est un groupe hydroxy, un groupe O-C (O)-R²⁰ ou OR²¹, où R²⁰ et R²¹ représentent chacun un groupe alkyle en C₁₋₁₂, un groupe cycloalkyle en C₃₋₈, un groupe aryle ayant 6 à 15 atomes de carbone, qui peut être substitué, ou un groupe aryl (alkyle en C₁₋₄), qui comprend en tout 7 à 15 atomes de carbone et peut être substitué sur le radical aryle,
R³ est un radical de formule CnF₂ₙ₊₁, dans laquelle n = 1, 2, 3, 4, 5 ou 6,
R⁴ et R⁵ représentent chacun un atome d'hydrogène, ou forment ensemble une double liaison ou un pont méthylène,
R⁵ et R⁶ représentent chacun un atome d'hydrogène, ou forment ensemble une double liaison ou un pont méthylène,
STEROID désigne un système cyclique de type stéroïde ABC, ayant les formules partielles A, B, C, D, E et F, 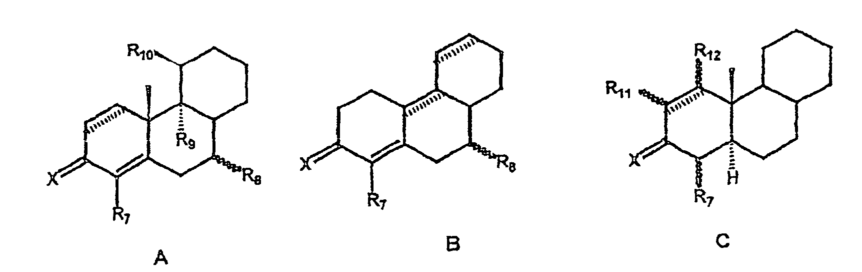 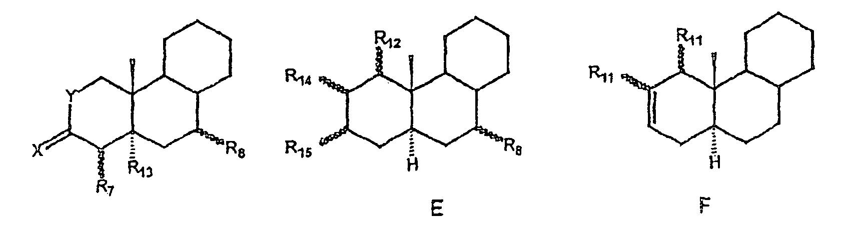 où l'on peut avoir en A et en C en position 1,2 une double liaison supplémentaire, et en B, en position 8,9 et en position 11,12, une ou deux doubles liaisons supplémentaires,
R⁷ est un atome d'hydrogène, un atome d'halogène, un groupe hydroxyle ou un groupe trifluorométhyle,
X est un atome d'hydrogène ou représente deux atomes d'hydrogène,
R⁸ est un atome d'hydrogène, un groupe méthyle ou éthyle,
R⁹ est un atome d'hydrogène ou un atome d'halogène, ou ensemble avec R¹⁰ forme une double liaison,
R¹⁰ est un atome d'hydrogène, un groupe hydroxyle, un groupe méthyle ou éthyle, ou ensemble avec R⁹ forme une double liaison,
R¹¹ est un atome d'hydrogène, un groupe alkyle en C₁₋₄, un groupe nitrile, un groupe hydroxyméthylène ou formyle,
R¹² est un atome d'hydrogène, un groupe alkyle en C₁₋₄ ou un groupe nitrile,
R¹¹ et R¹², outre les significations mentionnées ci-dessus, représentent ensemble un pont méthylène,
R¹³ est un atome d'hydrogène, ou ensemble avec R⁷ forme une double liaison,
R¹⁴ et R¹⁵ forment ensemble une double liaison, un noyau oxiranne, un noyau thiiranne, un noyau [2,3c]oxadiazole, un noyau [3,2c]isoxazole ou un noyau [3,2c]pyrazole,
Y est un atome d'oxygène ou d'azote,
où les lignes ondulées, au niveau de R⁷, R⁸, R¹¹, R¹², R¹³, R¹⁴ et R¹⁵, indiquent que ces substituants peuvent être en position α ou β, à l'exception des composés suivants :
17β-hydroxy-17α-trifluorométhyl-androst-4-ène-3-one,
17β-hydroxy-17α-trifluorométhyl-oestr-4-ène-3-one,
17β-hydroxy-17α-trifluorométhyl-oestr-4,9-diène-3-one,
17β-hydroxy-17α-trifluorométhyl-oestr-4,9,11-triène-3-one,
13-éthyl-17β-hydroxy-17α-trifluorométhyl-gone-4-ène-3-one,
13-éthyl-17β-hydroxy-17α-trifluorométhyl-gone-4,9-diène-3-one et
13-éthyl-17β-hydroxy-17α-trifluorométhyl-gone-4,9,11-triène-3-one,
pour préparer des médicaments destinés à la régulation de la fertilité chez l'homme, à l'hormonothérapie substitutive (HTS) chez l'homme et la femme, ou au traitement de maladies d'origine hormonale chez l'homme ou la femme, telles par exemple que l'endométriose, le cancer du sein ou l'hypogonadisme.
